# EUROPEAN PATENT SPECIFICATION

(11) **EP 4 218 795 B1**
(45) Date of publication and mention of the grant of the patent: **04.11.2026**
(21) Application number: 23157504.4
(22) Date of filing: 28.09.2017
(51) Int. Cl.: A61K 38/29, A61K 9/00, A61K 47/34, A61P 5/18, A61P 5/20

(54) **PTH COMPOUNDS WITH LOW PEAK-TO-TROUGH RATIOS**
PTH-VERBINDUNGEN MIT NIEDRIGEM SPITZE-ZU-TIEFPUNKT-VERHÄLTNIS
COMPOSES PTH AYANT DE FAIBLES RAPPORTS ENTRE LA CONCENTRATION MAXIMALE ET MINIMALE

(30) Priority: 29.09.2016 EP 16191454; 13.02.2017 EP 17155846
(43) Date of publication of application: 02.08.2023
(62) Document number(s) of the earlier application(s) in accordance with Art. 76 EPC: 17781427.4 / 3 518 961
(73) Proprietor: Ascendis Pharma Bone Diseases A/S, 2900 Hellerup (DK)
(72) Inventor: Sprogøe, Kennett, 2900 Hellerup (DK); Holten-Andersen, Lars, 2900 Hellerup (DK); Karpf, David Brian, Palo Alto, 94304 (US)
(74) Representative: Büchel, Edwin

(56) References cited:
- WO-A1-2011/012719
- WO-A1-2011/012721
- WO-A1-2011/012723
- WO-A1-2011/042450
- WO-A1-2011/089214
- WO-A1-2011/089215
- WO-A1-2011/089216
- WO-A1-2013/024049
- WO-A1-2013/024051
- WO-A1-2013/024053
- WO-A1-2013/053856
- WO-A1-2014/056915
- WO-A1-2014/056923
- WO-A1-2014/056926
- WO-A1-2014/086961
- WO-A1-2014/173759
- WO-A1-2015/052155
- WO-A1-2016/020373
- WO-A1-2016/110577

## Description

The present invention relates to a pharmaceutical composition comprising a PTH compound as defined in claim 1.

Hypoparathyroidism is a rare endocrine disorder of calcium and phosphate metabolism that most often arises as a result of parathyroid gland damage or removal during surgery of the thyroid gland. Hypoparathyroidism is unusual among endocrine disorders in that it has not been treated, until recently, by replacement with the missing hormone, parathyroid hormone, or PTH. Conventional therapy for hypoparathyroidism involves large doses of vitamin D and oral calcium supplementation, which, although often effective, is associated with marked swings in blood Ca²⁺ resulting in hypercalcemia and hypocalcemia, excess urinary calcium excretion, and nephrocalcinosis.

Calcium is the most abundant mineral in the human body, and its tight regulation is required for many critical biological functions, such as bone mineralization, muscle contraction, nerve conduction, hormone release, and blood coagulation. It is particularly important to maintain calcium concentration as stable as possible, because of the high sensitivity of a variety of cell systems or organs, including the central nervous system, muscle, and exo/endocrine glands, to small variations in Ca²⁺. PTH is a major regulator of calcium homeostasis.

The inappropriately low PTH level in relation to serum Ca²⁺ concentration, characteristic of hypoparathyroidism, leads to decreased renal tubular reabsorption of Ca²⁺ and simultaneously, to increased renal tubular reabsorption of phosphate. Thus, the main biochemical abnormalities of hypoparathyroidism are hypocalcemia and hyperphosphatemia. Clinical features of the disease include symptoms of hypocalcemia, such as perioral numbness, paresthesias, and carpal/pedal muscle spasms. Laryngeal spasm, tetany, and seizures are serious and potentially life-threatening complications. Hyperphosphatemia and an elevated calcium x phosphate product contributes to ectopic deposition of insoluble calcium phosphate complexes in soft tissues, including vasculature, brain, kidneys, and other organs.

Standard therapy of hypoparathyroidism is oral calcium and vitamin D supplementation. The goals of therapy are to a) ameliorate symptoms of hypocalcemia; b) maintain fasting serum calcium within or slightly below to the low-normal range; c) maintain fasting serum phosphorus within the high normal range or only slightly elevated; d) avoid or minimize hypercalciuria; e) maintain a calcium-phosphate product at levels well below the upper limit of normal and f) avoid ectopic calcification of the kidney (stones and nephrocalcinosis) and other soft tissues.

Several concerns arise with prolonged use of calcium and active vitamin D in large doses, particularly with regard to hypercalciuria, kidney stones, nephrocalcinosis and ectopic soft tissue calcification. In addition, conventional therapy with calcium and active vitamin D does not alleviate quality of life complaints nor does it reverse abnormalities in bone remodeling characteristic of the disease. In short, there is a high need for improved therapies for hypoparathyroidism.

In 2015, Natpara, PTH(1-84), was approved for once-daily subcutaneous injection as an adjunct to vitamin D and calcium in patients with hypoparathyroidism. Natpara, PTH(1-84), was approved to control hypocalcemia based on a pivotal trial demonstrating that 42 percent of PTH(1-84) treated participants achieved normal blood calcium levels on reduced doses of calcium supplements and active forms of vitamin D, compared to 3 percent of placebo-treated participants. Following a time course in which serum calcium was monitored after injection, 71 percent of patients treated with PTH(1-84) developed hypercalcemia at one or more measurements during a 24-hour period. PTH(1-84) reduced urinary calcium excretion 2-8 hours after injection but over the 24-hour period, urinary calcium excretion did not change. Similarly, urinary phosphate excretion increased only during the first 8 hours after PTH(1-84) injection.

While this represents an important advance in the treatment of the disease, Natpara has not demonstrated an ability to reduce incidences of hypercalcemia (elevated serum calcium levels), hypocalcemia (low serum calcium), or hypercalciuria (elevated urinary calcium) relative to conventional therapy in treated patients.

As such, there is a high need for improved PTH based therapies for hypoparathyroidism.

PTH(1-34), or teriparatide, was approved by the FDA in 2002 for the treatment of osteoporosis. Despite not being approved for this indication, PTH(1-34) has historically been used for treatment of hypoparathyroidism with patients receiving twice- or thrice-daily injections. To facilitate more physiological PTH levels, clinical studies have been conducted with PTH(1-34) administered by pump delivery in comparison with twice-daily injections. Over 6-months, pump delivery produced normal, steady state calcium levels with minimal fluctuation and avoided the rise in serum and urine calcium levels that are evident soon after PTH injection. The marked reduction in urinary calcium excretion when PTH(1-34) is administered by pump may indicate that PTH must be continuously exposed to the renal tubule for the renal calcium-conserving effects to be realized. Pump delivery of PTH(1-34) achieved simultaneous normalization of markers of bone turnover, serum calcium, and urine calcium excretion. These results were achieved with a lower daily PTH(1-34) dose and a reduced need for magnesium supplementation compared with the twice daily PTH(1-34) injection regimen.

However, continuous pump therapy is inconvenient and challenging for patients, and it is an object of the current invention to provide for a more convenient therapeutic option of providing continuous exposure to PTH.

Long-term bolus administration of PTH is associated with a progressive cortical bone loss due to increased bone metabolism. In a 6-year follow-up of patients treated with PTH(1-84) (Rubin, JCEM 2016) bone turnover markers remained greater than pretreatment values, peaking at the early years after PTH(1-84) initiation and declining thereafter but remaining significantly higher than baseline values by year 6. Bone mineral density (BMD) by dual X-ray absorptometry (DXA) was consistent with known site-specific effects of PTH, namely increases in lumbar spine and declines in the distal 1/3 radius (i/3 radius). The decrease observed at the i/3 radius is consistent with the known effects of intermittent PTH to increase cortical porosity and endosteal resorption.

It is an object of this invention to provide for a method of intermittently administering PTH, with lower elevation of bone turnover markers than currently applied PTH therapies. Preferably intermittent means with daily intervals, or alternatively or more preferably with weekly intervals.

In the preclinical development program of both Forteo, PTH(1-34) and Natpara, PTH(1-84) a dose dependent increase in osteosarcoma rate was observed in rats treated with daily injections of the PTH compound. In the Natpara study, dosing of the high dose rats were discontinued due to excessive deaths in this group, primarily from metastatic osteosarcoma.

As such it is an object of this invention to provide for an intermittent PTH replacement therapy that provides for symptom control with a lower administered dose. Preferably intermittent means with daily intervals, or alternatively or more preferably with weekly intervals.

In summary, there is a need for a more convenient and safer treatment of hypoparathyroidism with reduced side-effects.

It is therefore an object of the present invention to at least partially overcome the shortcomings described above.

This object is achieved with a pharmaceutical composition comprising a PTH compound as defined in claim 1.

It was surprisingly found that such PTH compound is capable of achieving a stable plasma profile of PTH which ensures physiological serum and urinary calcium levels or even reduced urinary calcium levels comparable to those measured in healthy subjects.

Within the present invention the terms are used having the meaning as follows.

As used herein the term "injection interval" refers to the time between two consecutive administrations of the pharmaceutical composition of the present invention.

As used herein the term "steady-state" refers to a state achieved after a multitude of constant doses, such as after 3, 4, 5, 6, 7 or more constant doses, of a medicament have been administered to a patient with constant time intervals between two consecutive administration, which state is characterized in that the plasma levels of the PTH compound, if the PTH compound does not release PTH, i.e. is a stable PTH compound, or of released PTH, if the PTH compound is a controlled-release PTH compound, at the beginning and at the end of an interval vary by no more than 10%, preferably by no more than 7.5%, even more preferably by no more than 5%, even more preferably by no more than 4% and most preferably by no more than 3%. The term "beginning of an interval" refers to the time point at which a dose is administered and the term "end of an interval" refers to the time point at which the next dose is administered.

As used herein the term "peak to trough ratio" refers to the ratio between the highest plasma concentration and the lowest plasma concentration of the PTH compound at steady state. If the PTH compound does not release PTH, i.e. is a stable PTH compound, or of released PTH, if the PTH compound is a controlled-release PTH compound, within the time period between two consecutive administrations to a non-human primate, preferably to a cynomolgus monkey. However, as there is a good correlation between free PTH and total PTH (i.e. the sum of PTH still comprised in the PTH compound and released PTH), so total PTH provides a sufficiently good approximation. The time period between two consecutive administrations is also referred to as "injection interval", "administration interval" or "interval".

As used herein the term "controlled-release PTH compound" refers to any compound, conjugate, crystal or admixture that comprises at least one PTH molecule or PTH moiety and from which the at least one PTH molecule or PTH moiety is released with a release half-life of at least 12 hours.

As used herein the terms "release half-life" and "half-life" refer to the time required under physiological conditions (i.e. aqueous buffer, pH 7.4, 37°C) until half of all PTH or PTH moieties, respectively, comprised in a controlled-release PTH compound are released from said controlled-release PTH compound.

As used herein the term "stable PTH compound" refers to any covalent conjugate of at least one PTH moiety to another moiety, wherein the at least one PTH moiety is connected to said other moiety through a stable linkage.

As used herein the term "PTH" refers all PTH polypeptides, preferably from mammalian species, more preferably from human and mammalian species, more preferably from human and murine species, as well as their variants, analogs, orthologs, homologs, and derivatives and fragments thereof, that are characterized by raising serum calcium and renal phosphorus excretion, and lowering serum phosphorus and renal calcium excretion. The term "PTH" also refers to all PTH-related polypeptides (PTHrP), such as the polypeptide of SEQ ID NO:121, that bind to and activate the common PTH/PTHrP1 receptor. Preferably, the term "PTH" refers to the PTH polypeptide of SEQ ID NO:51 as well as its variants, homologs and derivatives exhibiting essentially the same biological activity, i.e. raising serum calcium and renal phosphorus excretion, and lowering serum phosphorus and renal calcium excretion.

Preferably, the term "PTH" refers to the following polypeptide sequences:
SEQ ID NO:1 (PTH 1-84) 
SVSEIQLMHNLGKHLNSMERVEWLRKKLQDVHNFVALGAPLAPRDAGSQRPRKKED
NVLVESHEKSLGEADKADVNVLTKAKSQ
SEQ ID NO:2 (PTH 1-83) 
SVSEIQLMHNLGKHLNSMERVEWLRKKLQDVHNFVALGAPLAPRDAGSQRPRKKED
NVLVESHEKSLGEADKADVNVLTKAKS
SEQ ID NO:3 (PTH 1-82) 
SVSEIQLMHNLGKHLNSMERVEWLRKKLQDVHNFVALGAPLAPRDAGSQRPRKKED
NVLVESHEKSLGEADKADVNVLTKAK
SEQ ID NO:4 (PTH 1-81) 
SVSEIQLMHNLGKHLNSMERVEWLRKKLQDVHNFVALGAPLAPRDAGSQRPRKKED
NVLVESHEKSLGEADKADVNVLTKA
SEQ ID NO:5 (PTH 1-80) 
SVSEIQLMHNLGKHLNSMERVEWLRKKLQDVHNFVALGAPLAPRDAGSQRPRKKED
NVLVESHEKSLGEADKADVNVLTK
SEQ ID NO:6 (PTH 1-79) 
SVSEIQLMHNLGKHLNSMERVEWLRKKLQDVHNFVALGAPLAPRDAGSQRPRKKED
NVLVESHEKSLGEADKADVNVLT
SEQ ID NO:7 (PTH 1-78) 
SVSEIQLMHNLGKHLNSMERVEWLRKKLQDVHNFVALGAPLAPRDAGSQRPRKKED
NVLVESHEKSLGEADKADVNVL
SEQ ID NO:8 (PTH 1-77) 
SVSEIQLMHNLGKHLNSMERVEWLRKKLQDVHNFVALGAPLAPRDAGSQRPRKKED
NVLVESHEKSLGEADKADVNV
SEQ ID NO:9 (PTH 1-76) 
SVSEIQLMHNLGKHLNSMERVEWLRKKLQDVHNFVALGAPLAPRDAGSQRPRKKED
NVLVESHEKSLGEADKADVN
SEQ ID NO:10 (PTH 1-75) 
SVSEIQLMHNLGKHLNSMERVEWLRKKLQDVHNFVALGAPLAPRDAGSQRPRKKED
NVLVESHEKSLGEADKADV
SEQ ID NO:11 (PTH 1-74) 
SVSEIQLMHNLGKHLNSMERVEWLRKKLQDVHNFVALGAPLAPRDAGSQRPRKKED
NVLVESHEKSLGEADKAD
SEQ ID NO:12 (PTH 1-73) 
SVSEIQLMHNLGKHLNSMERVEWLRKKLQDVHNFVALGAPLAPRDAGSQRPRKKED
NVLVESHEKSLGEADKA
SEQ ID NO:13 (PTH 1-72) 
SVSEIQLMHNLGKHLNSMERVEWLRKKLQDVHNFVALGAPLAPRDAGSQRPRKKED
NVLVESHEKSLGEADK
SEQ ID NO:14 (PTH 1-71) 
SVSEIQLMHNLGKHLNSMERVEWLRKKLQDVHNFVALGAPLAPRDAGSQRPRKKED
NVLVESHEKSLGEAD
SEQ ID NO:15 (PTH 1-70) 
SVSEIQLMHNLGKHLNSMERVEWLRKKLQDVHNFVALGAPLAPRDAGSQRPRKKED
NVLVESHEKSLGEA
SEQ ID NO:16 (PTH 1-69) 
SVSEIQLMHNLGKHLNSMERVEWLRKKLQDVHNFVALGAPLAPRDAGSQRPRKKED
NVLVESHEKSLGE
SEQ ID NO:17 (PTH 1-68) 
SVSEIQLMHNLGKHLNSMERVEWLRKKLQDVHNFVALGAPLAPRDAGSQRPRKKED
NVLVESHEKSLG
SEQ ID NO:18 (PTH 1-67) 
SVSEIQLMHNLGKHLNSMERVEWLRKKLQDVHNFVALGAPLAPRDAGSQRPRKKED
NVLVESHEKSL
SEQ ID NO:19 (PTH 1-66) 
SVSEIQLMHNLGKHLNSMERVEWLRKKLQDVHNFVALGAPLAPRDAGSQRPRKKED
NVLVESHEKS
SEQ ID NO:20 (PTH 1-65) 
SVSEIQLMHNLGKHLNSMERVEWLRKKLQDVHNFVALGAPLAPRDAGSQRPRKKED
NVLVESHEK
SEQ ID NO:21 (PTH 1-64) 
SVSEIQLMHNLGKHLNSMERVEWLRKKLQDVHNFVALGAPLAPRDAGSQRPRKKED
NVLVESHE
SEQ ID NO:22 (PTH 1-63) 
SVSEIQLMHNLGKHLNSMERVEWLRKKLQDVHNFVALGAPLAPRDAGSQRPRKKED
NVLVESH
SEQ ID NO:23 (PTH 1-62) 
SVSEIQLMHNLGKHLNSMERVEWLRKKLQDVHNFVALGAPLAPRDAGSQRPRKKED
NVLVES
SEQ ID NO:24 (PTH 1-61) 
SVSEIQLMHNLGKHLNSMERVEWLRKKLQDVHNFVALGAPLAPRDAGSQRPRKKED
NVLVE
SEQ ID NO:25 (PTH 1-60) 
SVSEIQLMHNLGKHLNSMERVEWLRKKLQDVHNFVALGAPLAPRDAGSQRPRKKED
NVLV
SEQ ID NO:26 (PTH 1-59) 
SVSEIQLMHNLGKHLNSMERVEWLRKKLQDVHNFVALGAPLAPRDAGSQRPRKKED
NVL
SEQ ID NO:27 (PTH 1-58) 
SVSEIQLMHNLGKHLNSMERVEWLRKKLQDVHNFVALGAPLAPRDAGSQRPRKKED
NV
SEQ ID NO:28 (PTH 1-57) 
SVSEIQLMHNLGKHLNSMERVEWLRKKLQDVHNFVALGAPLAPRDAGSQRPRKKED
N
SEQ ID NO:29 (PTH 1-56)
   SVSEIQLMHNLGKHLNSMERVEWLRKKLQDVHNFVALGAPLAPRDAGSQRPRKKED
SEQ ID NO:30 (PTH 1-55)
   SVSEIQLMHNLGKHLNSMERVEWLRKKLQDVHNFVALGAPLAPRDAGSQRPRKKE
SEQ ID NO:31 (PTH 1-54)
   SVSEIQLMHNLGKHLNSMERVEWLRKKLQDVHNFVALGAPLAPRDAGSQRPRKK
SEQ ID NO:32 (PTH 1-53)
   SVSEIQLMHNLGKHLNSMERVEWLRKKLQDVHNFVALGAPLAPRDAGSQRPRK
SEQ ID NO:33 (PTH 1-52)
   SVSEIQLMHNLGKHLNSMERVEWLRKKLQDVHNFVALGAPLAPRDAGSQRPR
SEQ ID NO:34 (PTH 1-51)
   SVSEIQLMHNLGKHLNSMERVEWLRKKLQDVHNFVALGAPLAPRDAGSQRP
SEQ ID NO:35 (PTH 1-50)
   SVSEIQLMHNLGKHLNSMERVEWLRKKLQDVHNFVALGAPLAPRDAGSQR
SEQ ID NO:36 (PTH 1-49)
   SVSEIQLMHNLGKHLNSMERVEWLRKKLQDVHNFVALGAPLAPRDAGSQ
SEQ ID NO:37 (PTH 1-48)
   SVSEIQLMHNLGKHLNSMERVEWLRKKLQDVHNFVALGAPLAPRDAGS
SEQ ID NO:38 (PTH 1-47)
   SVSEIQLMHNLGKHLNSMERVEWLRKKLQDVHNFVALGAPLAPRDAG
SEQ ID NO:39 (PTH 1-46)
   SVSEIQLMHNLGKHLNSMERVEWLRKKLQDVHNFVALGAPLAPRDA
SEQ ID NO:40 (PTH 1-45)
   SVSEIQLMHNLGKHLNSMERVEWLRKKLQDVHNFVALGAPLAPRD
SEQ ID NO:41 (PTH 1-44)
   SVSEIQLMHNLGKHLNSMERVEWLRKKLQDVHNFVALGAPLAPR
SEQ ID NO:42 (PTH 1-43)
   SVSEIQLMHNLGKHLNSMERVEWLRKKLQDVHNFVALGAPLAP
SEQ ID NO:43 (PTH 1-42)
   SVSEIQLMHNLGKHLNSMERVEWLRKKLQDVHNFVALGAPLA
SEQ ID NO:44 (PTH 1-41)
   SVSEIQLMHNLGKHLNSMERVEWLRKKLQDVHNFVALGAPL
SEQ ID NO:45 (PTH 1-40)
   SVSEIQLMHNLGKHLNSMERVEWLRKKLQDVHNFVALGAP
SEQ ID NO:46 (PTH 1-39)
   SVSEIQLMHNLGKHLNSMERVEWLRKKLQDVHNFVALGA
SEQ ID NO:47 (PTH 1-38)
   SVSEIQLMHNLGKHLNSMERVEWLRKKLQDVHNFVALG
SEQ ID NO:48 (PTH 1-37)
   SVSEIQLMHNLGKHLNSMERVEWLRKKLQDVHNFVAL
SEQ ID NO:49 (PTH 1-36)
   SVSEIQLMHNLGKHLNSMERVEWLRKKLQDVHNFVA
SEQ ID NO:50 (PTH 1-35)
   SVSEIQLMHNLGKHLNSMERVEWLRKKLQDVHNFV
SEQ ID NO:51 (PTH 1-34)
   SVSEIQLMHNLGKHLNSMERVEWLRKKLQDVHNF
SEQ ID NO:52 (PTH 1-33)
   SVSEIQLMHNLGKHLNSMERVEWLRKKLQDVHN
SEQ ID NO:53 (PTH 1-32)
   SVSEIQLMHNLGKIALNSMERVEWLRKKLQDVH
SEQ ID NO:54 (PTH 1-31)
   SVSEIQLMHNLGKHLNSMERVEWLRKKLQDV
SEQ ID NO:55 (PTH 1-30)
   SVSEIQLMHNLGKHLNSMERVEWLRKKLQD
SEQ ID NO:56 (PTH 1-29)
   SVSEIQLMHNLGKHLNSMERVEWLRKKLQ
SEQ ID NO:57 (PTH 1-28)
   SVSEIQLMHNLGKHLNSMERVEWLRKKL
SEQ ID NO:58 (PTH 1-27)
   SVSEIQLMHNLGKHLNSMERVEWLRKK
SEQ ID NO:59 (PTH 1-26)
   SVSEIQLMHNLGKHLNSMERVEWLRK
SEQ ID NO:60 (PTH 1-25)
   SVSEIQLMHNLGKHLNSMERVEWLR
SEQ ID NO:61 (amidated PTH 1-84)
   SVSEIQLMHNLGKHLNSMERVEWLRKKLQDVHNFVALGAPLAPRDAGSQRPRKKED NVLVESHEKSLGEADKADVNVLTKAKSQ; wherein the C-terminus is amidated
SEQ ID NO:62 (amidated PTH 1-83)
   SVSEIQLMHNLGKHLNSMERVEWLRKKLQDVHNFVALGAPLAPRDAGSQRPRKKED NVLVESHEKSLGEADKADVNVLTKAKS; wherein the C-terminus is amidated
SEQ ID NO:63 (amidated PTH 1-82)
   SVSEIQLMHNLGKHLNSMERVEWLRKKLQDVHNFVALGAPLAPRDAGSQRPRKKED NVLVESHEKSLGEADKADVNVLTKAK; wherein the C-terminus is amidated
SEQ ID NO:64 (amidated PTH 1-81)
   SVSEIQLMHNLGKHLNSMERVEWLRKKLQDVHNFVALGAPLAPRDAGSQRPRKKED NVLVESHEKSLGEADKADVNVLTKA; wherein the C-terminus is amidated
SEQ ID NO:65 (amidated PTH 1-80)
   SVSEIQLMHNLGKHLNSMERVEWLRKKLQDVHNFVALGAPLAPRDAGSQRPRKKED NVLVESHEKSLGEADKADVNVLTK; wherein the C-terminus is amidated
SEQ ID NO:66 (amidated PTH 1-79)
   SVSEIQLMHNLGKHLNSMERVEWLRKKLQDVHNFVALGAPLAPRDAGSQRPRKKED NVLVESHEKSLGEADKADVNVLT; wherein the C-terminus is amidated
SEQ ID NO:67 (amidated PTH 1-78)
   SVSEIQLMHNLGKHLNSMERVEWLRKKLQDVHNFVALGAPLAPRDAGSQRPRKKED NVLVESHEKSLGEADKADVNVL; wherein the C-terminus is amidated
SEQ ID NO:68 (amidated PTH 1-77)
   SVSEIQLMHNLGKHLNSMERVEWLRKKLQDVHNFVALGAPLAPRDAGSQRPRKKED NVLVESHEKSLGEADKADVNV; wherein the C-terminus is amidated
SEQ ID NO:69 (amidated PTH 1-76)
   SVSEIQLMHNLGKHLNSMERVEWLRKKLQDVHNFVALGAPLAPRDAGSQRPRKKED NVLVESHEKSLGEADKADVN; wherein the C-terminus is amidated
SEQ ID NO:70 (amidated PTH 1-75)
   SVSEIQLMHNLGKHLNSMERVEWLRKKLQDVHNFVALGAPLAPRDAGSQRPRKKED NVLVESHEKSLGEADKADV; wherein the C-terminus is amidated
SEQ ID NO:71 (amidated PTH 1-74)
   SVSEIQLMHNLGKBLNSMERVEWLRKKLQDVHNFVALGAPLAPRDAGSQRPRKKED NVLVESHEKSLGEADKAD; wherein the C-terminus is amidated
SEQ ID NO:72 (amidated PTH 1-73)
   SVSEIQLMHNLGKHLNSMERVEWLRKKLQDVHNFVALGAPLAPRDAGSQRPRKKED NVLVESHEKSLGEADKA; wherein the C-terminus is amidated
SEQ ID NO:73 (amidated PTH 1-72)
   SVSEIQLMHNLGKHLNSMERVEWLRKKLQDVHNFVALGAPLAPRDAGSQRPRKKED NVLVESHEKSLGEADK; wherein the C-terminus is amidated
SEQ ID NO:74 (amidated PTH 1-71)
   SVSEIQLMHNLGKHLNSMERVEWLRKKLQDVHNFVALGAPLAPRDAGSQRPRKKED NVLVESHEKSLGEAD; wherein the C-terminus is amidated
SEQ ID NO:75 (amidated PTH 1-70)
   SVSEIQLMHNLGKHLNSMERVEWLRKKLQDVHNFVALGAPLAPRDAGSQRPRKKED NVLVESHEKSLGEA; wherein the C-terminus is amidated
SEQ ID NO:76 (amidated PTH 1-69)
   SVSEIQLMHNLGKHLNSMERVEWLRKKLQDVHNFVALGAPLAPRDAGSQRPRKKED NVLVESHEKSLGE; wherein the C-terminus is amidated
SEQ ID NO:77 (amidated PTH 1-68)
   SVSEIQLMHNLGKHLNSMERVEWLRKKLQDVHNFVALGAPLAPRDAGSQRPRKKED NVLVESHEKSLG; wherein the C-terminus is amidated
SEQ ID NO:78 (amidated PTH 1-67)
   SVSEIQLMHNLGKHLNSMERVEWLRKKLQDVHNFVALGAPLAPRDAGSQRPRKKED NVLVESHEKSL; wherein the C-terminus is amidated
SEQ ID NO:79 (amidated PTH 1-66)
   SVSEIQLMHNLGKHLNSMERVEWLRKKLQDVHNFVALGAPLAPRDAGSQRPRKKED NVLVESHEKS; wherein the C-terminus is amidated
SEQ ID NO:80 (amidated PTH 1-65)
   SVSEIQLMHNLGKHLNSMERVEWLRKKLQDVHNFVALGAPLAPRDAGSQRPRKKED NVLVESHEK; wherein the C-terminus is amidated
SEQ ID NO:81 (amidated PTH 1-64)
   SVSEIQLMHNLGKHLNSMERVEWLRKKLQDVHNFVALGAPLAPRDAGSQRPRKKED NVLVESHE; wherein the C-terminus is amidated
SEQ ID NO:82 (amidated PTH 1-63)
   SVSEIQLMHNLGKHLNSMERVEWLRKKLQDVHNFVALGAPLAPRDAGSQRPRKKED NVLVESH; wherein the C-terminus is amidated
SEQ ID NO:83 (amidated PTH 1-62)
   SVSEIQLMHNLGKHLNSMERVEWLRKKLQDVHNFVALGAPLAPRDAGSQRPRKKED NVLVES; wherein the C-terminus is amidated
SEQ ID NO:84 (amidated PTH 1-61)
   SVSEIQLMHNLGKHLNSMERVEWLRKKLQDVHNFVALGAPLAPRDAGSQRPRKKED NVLVE; wherein the C-terminus is amidated
SEQ ID NO:85 (amidated PTH 1-60)
   SVSEIQLMHNLGKHLNSMERVEWLRKKLQDVHNFVALGAPLAPRDAGSQRPRKKED NVLV; wherein the C-terminus is amidated
SEQ ID NO:86 (amidated PTH 1-59)
   SVSEIQLMHNLGKHLNSMERVEWLRKKLQDVHNFVALGAPLAPRDAGSQRPRKKED NVL; wherein the C-terminus is amidated
SEQ ID NO:87 (amidated PTH 1-58)
   SVSEIQLMHNLGKHLNSMERVEWLRKKLQDVHNFVALGAPLAPRDAGSQRPRKKED NV; wherein the C-terminus is amidated
SEQ ID NO:88 (amidated PTH 1-57)
   SVSEIQLMHNLGKHLNSMERVEWLRKKLQDVHNFVALGAPLAPRDAGSQRPRKKED N; wherein the C-terminus is amidated
SEQ ID NO:89 (amidated PTH 1-56)
   SVSEIQLMHNLGKHLNSMERVEWLRKKLQDVHNFVALGAPLAPRDAGSQRPRKKED ; wherein the C-terminus is amidated
SEQ ID NO:90 (amidated PTH 1-55)
   SVSEIQLMHNLGKHLNSMERVEWLRKKLQDVHNFVALGAPLAPRDAGSQRPRKKE;
   wherein the C-terminus is amidated
SEQ ID NO:91 (amidated PTH 1-54)
   SVSEIQLMHNLGKHLNSMERVEWLRKKLQDVHNFVALGAPLAPRDAGSQRPRKK;
   wherein the C-terminus is amidated
SEQ ID NO:92 (amidated PTH 1-53)
   SVSEIQLMHNLGKHLNSMERVEWLRKKLQDVHNFVALGAPLAPRDAGSQRPRK;
   wherein the C-terminus is amidated
SEQ ID NO:93 (amidated PTH 1-52)
   SVSEIQLMHNLGKHLNSMERVEWLRKKLQDVHNFVALGAPLAPRDAGSQRPR;
   wherein the C-terminus is amidated
SEQ ID NO:94 (amidated PTH 1-51)
   SVSEIQLMHNLGKHLNSMERVEWLRKKLQDVHNFV ALGAPLAPRDAGSQRP;
   wherein the C-terminus is amidated
SEQ ID NO:95 (amidated PTH 1-50)
   SVSEIQLMHNLGKHLNSMERVEWLRKKLQDVHNFVALGAPLAPRDAGSQR; wherein
   the C-terminus is amidated
SEQ ID NO:96 (amidated PTH 1-49)
   SVSEIQLMHNLGKHLNSMERVEWLRKKLQDVHNFVALGAPLAPRDAGSQ; wherein
   the C-terminus is amidated
SEQ ID NO:97 (amidated PTH 1-48)
   SVSEIQLMHNLGKHLNSMERVEWLRKKLQDVHNFVALGAPLAPRDAGS; wherein the C-terminus is amidated
SEQ ID NO:98 (amidated PTH 1-47)
   SVSEIQLMHNLGKHLNSMERVEWLRKKLQDVHNFVALGAPLAPRDAG; wherein the C-terminus is amidated
SEQ ID NO:99 (amidated PTH 1-46)
   SVSEIQLMHNLGKHLNSMERVEWLRKKLQDVHNFVALGAPLAPRDA; wherein the C-terminus is amidated
SEQ ID NO:100 (amidated PTH 1-45)
   SVSEIQLMHNLGKHLNSMERVEWLRKKLQDVHNFVALGAPLAPRD; wherein the C-terminus is amidated
SEQ ID NO:101 (amidated PTH 1-44)
   SVSEIQLMHNLGKHLNSMERVEWLRKKLQDVHNFVALGAPLAPR; wherein the C-terminus is amidated
SEQ ID NO:102 (amidated PTH 1-43)
   SVSEIQLMHNLGKHLNSMERVEWLRKKLQDVHNFVALGAPLAP; wherein the C-terminus is amidated
SEQ ID NO:103 (amidated PTH 1-42)
   SVSEIQLMHNLGKHLNSMERVEWLRKKLQDVHNFV ALGAPLA; wherein the C-terminus is amidated
SEQ ID NO:104 (amidated PTH 1-41)
   SVSEIQLMHNLGKHLNSMERVEWLRKKLQDVHNFVALGAPL; wherein the C-terminus is amidated
SEQ ID NO:105 (amidated PTH 1-40)
   SVSEIQLMHNLGKHLNSMERVEWLRKKLQDVHNFVALGAP; wherein the C-terminus is amidated
SEQ ID NO:106 (amidated PTH 1-39)
   SVSEIQLMHNLGKHLNSMERVEWLRKKLQDVHNFVALGA; wherein the C-terminus is amidated
SEQ ID NO:107 (amidated PTH 1-38)
   SVSEIQLMHNLGKHLNSMERVEWLRKKLQDVHNFVALG; wherein the C-terminus is amidated
SEQ ID NO:108 (amidated PTH 1-37)
   SVSEIQLMHNLGKHLNSMERVEWLRKKLQDVHNFVAL; wherein the C-terminus is amidated
SEQ ID NO:109 (amidated PTH 1-36)
   SVSEIQLMHNLGKHLNSMERVEWLRKKLQDVHNFVA; wherein the C-terminus is amidated
SEQ ID NO:110 (amidated PTH 1-35)
   SVSEIQLMHNLGKHLNSMERVEWLRKKLQDVHNFV; wherein the C-terminus is amidated
SEQ ID NO:111 (amidated PTH 1-34)
   SVSEIQLMHNLGKHLNSMERVEWLRKKLQDVHNF; wherein the C-terminus is amidated
SEQ ID NO:112 (amidated PTH 1-33)
   SVSEIQLMHNLGKHLNSMERVEWLRKKLQDVHN; wherein the C-terminus is amidated
SEQ ID NO:113 (amidated PTH 1-32)
   SVSEIQLMHNLGKHLNSMERVEWLRKKLQDVH; wherein the C-terminus is amidated
SEQ ID NO:114 (amidated PTH 1-31)
   SVSEIQLMHNLGKHLNSMERVEWLRKKLQDV; wherein the C-terminus is amidated
SEQ ID NO:115 (amidated PTH 1-30)
   SVSEIQLMHNLGKHLNSMERVEWLRKKLQD; wherein the C-terminus is amidated
SEQ ID NO:116 (amidated PTH 1-29)
   SVSEIQLMHNLGKHLNSMERVEWLRKKLQ; wherein the C-terminus is amidated
SEQ ID NO:117 (amidated PTH 1-28)
   SVSEIQLMHNLGKHLNSMERVEWLRKKL; wherein the C-terminus is amidated
SEQ ID NO:118 (amidated PTH 1-27)
   SVSEIQLMHNLGKHLNSMERVEWLRKK; wherein the C-terminus is amidated
SEQ ID NO:119 (amidated PTH 1-26)
   SVSEIQLMHNLGKHLNSMERVEWLRK; wherein the C-terminus is amidated
SEQ ID NO:120 (amidated PTH 1-25)
   SVSEIQLMHNLGKHLNSMERVEWLR; wherein the C-terminus is amidated
SEQ ID NO:121 (PTHrP)

AVSEHQLLHDKGKSIQDLRRRFFLHHLIAEIHTAEIRATSEVSPNSKPSPNTKNHPVRF
GSDDEGRYLTQETNKVETYKEQPLKTPGKKKKGKPGKRKEQEKKKRRTRSAWLDS
GVTGSGLEGDHLSDTSTTSLELDSRRH

More preferably, the term "PTH" refers to the sequence of SEQ ID:NOs 47, 48, 49, 50, 51, 52, 53, 54, 55, 107, 108, 109, 110, 111, 112, 113, 114 and 115. Even more preferably, the term "PTH" refers to the sequence of SEQ ID:NOs 50, 51, 52, 110, 111 and 112. In a particularly preferred embodiment the term "PTH" refers to the sequence of SEQ ID NO:51.

As used herein, the term "PTH polypeptide variant" refers to a polypeptide from the same species that differs from a reference PTH or PTHrP polypeptide. Preferably, such reference is a PTH polypeptide sequence and has the sequence of SEQ ID NO:51. Generally, differences are limited so that the amino acid sequence of the reference and the variant are closely similar overall and, in many regions, identical. Preferably, PTH polypeptide variants are at least 70%, 80%, 90%, or 95% identical to a reference PTH or PTHrP polypeptide, preferably to the PTH polypeptide of SEQ ID NO:51. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino (N-terminal) or carboxy terminal (C-terminal) positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein. Preferably, the query sequence is the sequence of SEQ ID NO:51.

Such PTH polypeptide variants may be naturally occurring variants, such as naturally occurring allelic variants encoded by one of several alternate forms of a PTH or PTHrP occupying a given locus on a chromosome or an organism, or isoforms encoded by naturally occurring splice variants originating from a single primary transcript. Alternatively, a PTH polypeptide variant may be a variant that is not known to occur naturally and that can be made by mutagenesis techniques known in the art.

It is known in the art that one or more amino acids may be deleted from the N-terminus or C-terminus of a bioactive polypeptide without substantial loss of biological function. Such N- and/or C-terminal deletions are also encompassed by the term PTH polypeptide variant.

It is also recognized by one of ordinary skill in the art that some amino acid sequences of PTH or PTHrP polypeptides can be varied without significant effect of the structure or function of the polypeptide. Such mutants include deletions, insertions, inversions, repeats, and substitutions selected according to general rules known in the art so as to have little effect on activity. For example, guidance concerning how to make phenotypically silent amino acid substitutions is provided in Bowie et al. (1990), Science 247:1306-1310, wherein the authors indicate that there are two main approaches for studying the tolerance of the amino acid sequence to change.

The term PTH polypeptide also encompasses all PTH and PTHrP polypeptides encoded by PTH and PTHrP analogs, orthologs, and/or species homologs. It is also recognized by one of ordinary skill in the art that PTHrP and PTHrP analogs bind to activate the common PTH/PTHrP1 receptor, so the term PTH polypeptide also encompasses all PTHrP analogs. As used herein, the term "PTH analog" refers to PTH and PTHrP of different and unrelated organisms which perform the same functions in each organism but which did not originate from an ancestral structure that the organisms' ancestors had in common. Instead, analogous PTH and PTHrP arose separately and then later evolved to perform the same or similar functions. In other words, analogous PTH and PTHrP polypeptides are polypeptides with quite different amino acid sequences but that perform the same biological activity, namely raising serum calcium and renal phosphorus excretion, and lowering serum phosphorus and renal calcium excretion.

As used herein the term "PTH ortholog" refers to PTH and PTHrP within two different species which sequences are related to each other via a common homologous PTH or PTHrP in an ancestral species, but which have evolved to become different from each other.

As used herein, the term "PTH homolog" refers to PTH and PTHrP of different organisms which perform the same functions in each organism and which originate from an ancestral structure that the organisms' ancestors had in common. In other words, homologous PTH polypeptides are polypeptides with quite similar amino acid sequences that perform the same biological activity, namely raising serum calcium and renal phosphorus excretion, and lowering serum phosphorus and renal calcium excretion. Preferably, PTH polypeptide homologs may be defined as polypeptides exhibiting at least 40%, 50%, 60%, 70%, 80%, 90% or 95% identity to a reference PTH or PTHrP polypeptide, preferably the PTH polypeptide of SEQ ID NO:51.

Thus, a PTH polypeptide according to the invention may be, for example: (i) one in which at least one of the amino acids residues is substituted with a conserved or non-conserved amino acid residue, preferably a conserved amino acid residue, and such substituted amino acid residue may or may not be one encoded by the genetic code; and/or (ii) one in which at least one of the amino acid residues includes a substituent group; and/or (iii) one in which the PTH polypeptide is fused with another compound, such as a compound to increase the half-life of the polypeptide (for example, polyethylene glycol); and/or (iv) one in which additional amino acids are fused to the PTH polypeptide, such as an IgG Fc fusion region polypeptide or leader or secretory sequence or a sequence which is employed for purification of the above form of the polypeptide or a pre-protein sequence.

As used herein, the term "PTH polypeptide fragment" refers to any polypeptide comprising a contiguous span of a part of the amino acid sequence of a PTH or PTHrP polypeptide, preferably the polypeptide of SEQ ID NO:51.

More specifically, a PTH polypeptide fragment comprises at least 6, such as at least 8, at least 10 or at least 17 consecutive amino acids of a PTH or PTHrP polypeptide, more preferably of the polypeptide of SEQ ID NO:51. A PTH polypeptide fragment may additionally be described as sub-genuses of PTH or PTHrP polypeptides comprising at least 6 amino acids, wherein "at least 6" is defined as any integer between 6 and the integer representing the C-terminal amino acid of a PTH or PTHrP polypeptide, preferably of the polypeptide of SEQ ID No:51. Further included are species of PTH or PTHrP polypeptide fragments at least 6 amino acids in length, as described above, that are further specified in terms of their N-terminal and C-terminal positions. Also encompassed by the term "PTH polypeptide fragment" as individual species are all PTH or PTHrP polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of a PTH or PTHrP polypeptide, preferably the PTH polypeptide of SEQ ID:NO51, is included in the present invention.

The term "PTH" also includes poly(amino acid) conjugates which have a sequence as described above, but having a backbone that comprises both amide and non-amide linkages, such as ester linkages, like for example depsipeptides. Depsipeptides are chains of amino acid residues in which the backbone comprises both amide (peptide) and ester bonds. Accordingly, the term "side chain" as used herein refers either to the moiety attached to the alpha-carbon of an amino acid moiety, if the amino acid moiety is connected through amine bonds such as in polypeptides, or to any carbon atom-comprising moiety attached to the backbone of a poly(amino acid) conjugate, such as for example in the case of depsipeptides. Preferably, the term "PTH" refers to polypeptides having a backbone formed through amide (peptide) bonds.

As the term PTH includes the above-described variants, analogs, orthologs, homologs, derivatives and fragments of PTH and PTHrP, all references to specific positions within a reference sequence also include the equivalent positions in variants, analogs, orthologs, homologs, derivatives and fragments of a PTH or PTHrP moiety, even if not specifically mentioned.

As used herein the term "micelle" means an aggregate of amphiphilic molecules dispersed in a liquid colloid. In aqueous solution a typical micelle forms an aggregate with the hydrophilic moiety of the surfactant molecules facing the surrounding solvent and the hydrophobic moiety of the surfactant molecule facing inwards, also called "normal-phase micelle". "Invers micelles" have the hydrophilic moiety facing inwards and the hydrophobic moiety facing the surrounding solvent.

As used herein the term "liposome" refers to a vesicle, preferably a spherical vesicle, having at least one lipid bilayer. Preferably, liposomes comprise phospholipids, even more preferably phosphatidylcholine. The term "liposome" refers to various structures and sizes, such as, for example, to multilamellar liposome vesicles (MLV) having more than one concentric lipid bilayer with an average diameter of 100 to 1000 nm, small unilamellar liposome vesicles (SUV) having one lipid bilayer and an average diameter of 25 to 100 nm, large unilamellar liposome vesicles (LUV) having one lipid bilayer and an average diameter of about 1000 µm and giant unilamellar vesicles (GUV) having one lipid bilayer and an average diameter of 1 to 100 µm. The term "liposome" also includes elastic vesicles such as transferosomes and ethosomes, for example.

As used herein the term "aquasome" refers to spherical nanoparticles having a diameter of 60 to 300 nm that comprise at least three layers of self-assembled structure, namely a solid phase nanocrystalline core coated with an oligomeric film to which drug molecules are adsorbed with or without modification of the drug.

As used herein the term "ethosome" refers to lipid vesicles comprising phospholipids and ethanol and/or isopropanol in relatively high concentration and water, having a size ranging from tens of nanometers to micrometers.

As used herein the term "LeciPlex" refers to positively charged phospholipid-based vesicular system which comprises soy PC, a cationic agent, and a bio-compatible solvent like PEG 300, PEG 400, diethylene glycol monoethyl ether, tetrahydrofurfuryl alcohol polyethylene glycol ether or 2-pyrrolidoneor N-methyl-2-pyrrolidone.

As used herein the term "niosome" refers to unilamellar or multilamellar vesicles comprising non-ionic surfactants.

As used herein the term "pharmacosome" refers to ultrafine vesicular, micellar or hexagonal aggregates from lipids covalently bound to biologically active moieties.

As used herein the term "proniosome" refers to dry formulations of surfactant-coated carrier which on rehydration and mild agitation gives niosomes.

As used herein the term "polymersome" refers to an artificial spherical vesicle comprising a membrane formed from amphiphilic synthetic block copolymers and may optionally comprise an aqueous solution in its core. A polymersome has a diameter ranging from 50 nm to 5 µm and larger. The term also includes syntosomes, which are polymersomes engineered to comprise channels that allow certain chemicals to pass through the membrane into or out of the vesicle.

As used herein the term "sphingosome" refers to a concentric, bilayered vesicle in which an aqueous volume is entirely enclosed by a membranous lipid bilayer mainly composed of natural or synthetic sphingolipid.

As used herein the term "transferosome" refers to ultraflexible lipid vesicles comprising an aqueous core that are formed from a mixture of common polar and suitable edge-activated lipids which facilitate the formation of highly curved bilayers which render the transferosome highly deformable.

As used herein the term "ufasome" refers to a vesicle comprising unsaturated fatty acids.

As used herein the term "polypeptide" refers to a peptide comprising up to and including 50 amino acid monomers.

As used herein the term "protein" refers to a peptide of more than 50 amino acid residues. Preferably a protein comprises at most 20000 amino acid residues, such as at most 15000 amino acid residues, such as at most 10000 amino acid residues, such as at most 5000 amino acid residues, such as at most 4000 amino acid residues, such as at most 3000 amino acid residues, such as at most 2000 amino acid residues, such as at most 1000 amino acid residues.

As used herein the term "physiological conditions" refers to an aqueous buffer at pH 7.4, 37°C.

As used herein the term "pharmaceutical composition" refers to a composition containing one or more active ingredients, such as for example at least one controlled-release PTH compounds, and one or more excipients, as well as any product which results, directly or indirectly, from combination, complexation or aggregation of any two or more of the ingredients of the composition, or from dissociation of one or more of the ingredients, or from other types of reactions or interactions of one or more of the ingredients. Accordingly, the pharmaceutical compositions of the present invention encompass any composition made by admixing one or more controlled-release PTH compound and a pharmaceutically acceptable excipient.

As used herein the term "liquid composition" refers to a mixture comprising water-soluble PTH compound, preferably water-soluble controlled-release PTH compound, and one or more solvents, such as water.

The term "suspension composition" relates to a mixture comprising water-insoluble PTH compound, preferably water-insoluble controlled-release PTH compound, and one or more solvents, such as water.

As used herein, the term "dry composition" means that a pharmaceutical composition is provided in a dry form. Suitable methods for drying are spray-drying and lyophilization, i.e. freeze-drying. Such dry composition of prodrug has a residual water content of a maximum of 10 %, preferably less than 5% and more preferably less than 2%, determined according to Karl Fischer. Preferably, the pharmaceutical composition of the present invention is dried by lyophilization.

The term "drug" as used herein refers to a substance, such as PTH, used in the treatment, cure, prevention, or diagnosis of a disease or used to otherwise enhance physical or mental well-being. If a drug is conjugated to another moiety, the moiety of the resulting product that originated from the drug is referred to as "biologically active moiety".

As used herein the term "prodrug" refers to a conjugate in which a biologically active moiety is reversibly and covalently connected to a specialized protective group through a reversible linker moiety, also referred to as "reversible prodrug linker moiety", which comprises a reversible linkage with the biologically active moiety and wherein the specialized protective group alters or eliminates undesirable properties in the parent molecule. This also includes the enhancement of desirable properties in the drug and the suppression of undesirable properties. The specialized non-toxic protective group is referred to as "carrier". A prodrug releases the reversibly and covalently bound biologically active moiety in the form of its corresponding drug. In other words, a prodrug is a conjugate comprising a biologically active moiety which is covalently and reversibly conjugated to a carrier moiety via a reversible prodrug linker moiety, which covalent and reversible conjugation of the carrier to the reversible prodrug linker moiety is either directly or through a spacer. Such conjugate releases the formerly conjugated biologically active moiety in the form of a free drug.

As used herein the term "reversible prodrug linker moiety is a spacer moiety that connects a biologically active moiety, such as a PTH moiety, to a carrier moiety, either directly or through a further spacer moiety and wherein the linkage between the reversible prodrug linker moiety and the biologically active moiety is reversible. Preferably, the linkage between the carrier moiety and the reversible prodrug linker moiety is a stable.

A "biodegradable linkage" or a "reversible linkage" is a linkage that is hydrolytically degradable, i.e. cleavable, in the absence of enzymes under physiological conditions (aqueous buffer at pH 7.4, 37°C) with a half-life ranging from one hour to three months, preferably from one hour to two months, even more preferably from one hour to one month, even more preferably from one hour to three weeks, most preferably from one hour to two weeks. Accordingly, a "stable linkage" is a linkage having a half-life under physiological conditions (aqueous buffer at pH 7.4, 37°C) of more than three months.

As used herein, the term "traceless prodrug linker" means a reversible prodrug linker, i.e. a linker moiety reversibly and covalently connecting the biologically active moiety with the carrier, which upon cleavage releases the drug in its free form. As used herein, the term "free form" of a drug means the drug in its unmodified, pharmacologically active form.

As used herein, the term "excipient" refers to a diluent, adjuvant, or vehicle with which the therapeutic, such as a drug or prodrug, is administered. Such pharmaceutical excipient can be sterile liquids, such as water and oils, including those of petroleum, animal, vegetable or synthetic origin, including peanut oil, soybean oil, mineral oil and sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water andethanol. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(N-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders and sustained-release formulations. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose and magnesium carbonate. Such compositions will contain a therapeutically effective amount of the drug or biologically active moiety, together with a suitable amount of excipient so as to provide the form for proper administration to the patient. The formulation should suit the mode of administration.

As used herein, the term "reagent" means a chemical compound which comprises at least one functional group for reaction with the functional group of another chemical compound or drug. It is understood that a drug comprising a functional group (such as a primary or secondary amine or hydroxyl functional group) is also a reagent.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X-", whereas each "-" indicates attachment to another moiety. Accordingly, a biologically active moiety is released from a prodrug as a drug.

It is understood that if the sequence or chemical structure of a group of atoms is provided which group of atoms is attached to two moieties or is interrupting a moiety, said sequence or chemical structure can be attached to the two moieties in either orientation, unless explicitly stated otherwise. For example, a moiety "-C(O)N(R¹)- " can be attached to two moieties or interrupting a moiety either as "-C(O)N(R¹)- " or as "-N(R¹)C(O)- ". Similarly, a moiety 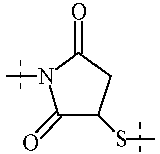 can be attached to two moieties or can interrupt a moiety either as 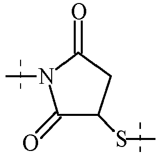 or as

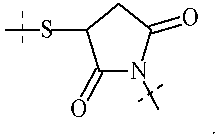

As used herein, the term "functional group" means a group of atoms which can react with other groups of atoms. Functional groups include but are not limited to the following groups: carboxylic acid (-(C=O)OH), primary or secondary amine (-NH₂, -NH-), maleimide, thiol (-SH), sulfonic acid (-(O=S=O)OH), carbonate, carbamate (-O(C=O)N<), hydroxyl (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, sulfonamides, sulfuric acid, vinyl sulfone, vinyl ketone, diazoalkane, oxirane, and aziridine.

In case the PTH compound, preferably controlled-release PTH compound, of the present invention comprise one or more acidic or basic groups, the invention also comprises their corresponding pharmaceutically or toxicologically acceptable salts, in particular their pharmaceutically utilizable salts. Thus, the PTH compound, preferably the controlled-release PTH compound, of the present invention comprising acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. PTH compound, preferably controlled-release PTH compound, of the present invention comprising one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. For the person skilled in the art further methods are known for converting the basic group into a cation like the alkylation of an amine group resulting in a positively-charge ammonium group and an appropriate counterion of the salt. If the PTH compound, preferably the controlled-release PTH compound, of the present invention simultaneously comprise acidic and basic groups, the invention also includes, in addition to the salt forms mentioned, inner salts or betaines (zwitterions). The respective salts can be obtained by customary methods which are known to the person skilled in the art like, for example by contacting these compounds with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means a substance that does cause harm when administered to a patient and preferably means approved by a regulatory agency, such as the EMA (Europe) and/or the FDA (US) and/or any other national regulatory agency for use in animals, preferably for use in humans.

As used herein the term "about" in combination with a numerical value is used to indicate a range ranging from and including the numerical value plus and minus no more than 10% of said numerical value, more preferably no more than 8% of said numerical value, even more preferably no more than 5% of said numerical value and most preferably no more than 2% of said numerical value. For example, the phrase "about 200" is used to mean a range ranging from and including 200 +/- 10%, i.e. ranging from and including 180 to 220; preferably 200 +/-8%, i.e. ranging from and including 184 to 216; even more preferably ranging from and including 200 +/-5%, i.e. ranging from and including 190 to 210; and most preferably 200 +/-2%, i.e. ranging from and including 196 to 204. It is understood that a percentage given as "about 20%" does not mean "20% +/- 10%", i.e. ranging from and including 10 to 30%, but "about 20%" means ranging from and including 18 to 22%, i.e. plus and minus 10% of the numerical value which is 20.

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. It is understood that a polymer may also comprise one or more other chemical groups and/or moieties, such as, for example, one or more functional groups. Preferably, a soluble polymer has a molecular weight of at least 0.5 kDa, e.g. a molecular weight of at least 1 kDa, a molecular weight of at least 2 kDa, a molecular weight of at least 3 kDa or a molecular weight of at least 5 kDa. If the polymer is soluble, it preferable has a molecular weight of at most 1000 kDa, such as at most 750 kDa, such as at most 500 kDa, such as at most 300 kDa, such as at most 200 kDa, such as at most 100 kDa. It is understood that for insoluble polymers, such as hydrogels, no meaningful molecular weight ranges can be provided. It is understood that also a protein is a polymer in which the amino acids are the repeating structural units, even though the side chains of each amino acid may be different.

As used herein, the term "polymeric" means a reagent or a moiety comprising one or more polymers or polymer moieties. A polymeric reagent or moiety may optionally also comprise one or more other moiety/moieties, which are preferably selected from the group consisting of:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 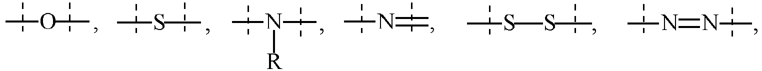 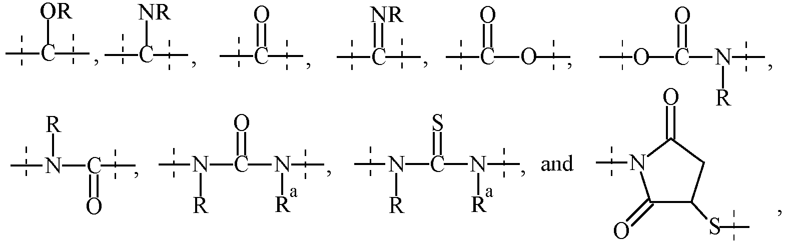 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers, i.e. to the arithmetic mean of the molecular weight of the polymer or polymeric moiety and the arithmetic mean of the number of monomers of the polymer or polymeric moiety.

Accordingly, in a polymeric moiety comprising "x" monomer units any integer given for "x" therefore corresponds to the arithmetic mean number of monomers. Any range of integers given for "x" provides the range of integers in which the arithmetic mean numbers of monomers lies. An integer for "x" given as "about x" means that the arithmetic mean numbers of monomers lies in a range of integers of x +/- 10%, preferably x +/- 8%, more preferably x +/- 5% and most preferably x +/- 2%.

As used herein, the term "number average molecular weight" means the ordinary arithmetic mean of the molecular weights of the individual polymers.

As used herein the term "water-soluble" with reference to a carrier means that when such carrier is part of PTH compound, preferably the controlled-release PTH compound, of the present invention at least 1 g of the PTH compound, preferably controlled-release PTH compound, comprising such water-soluble carrier can be dissolved in one liter of water at 20°C to form a homogeneous solution. Accordingly, the term "water-insoluble" with reference to a carrier means that when such carrier is part of a PTH compound, preferably controlled-release PTH compound, of the present invention less than 1 g of the PTH compound, preferably controlled-release PTH compound, comprising such water-insoluble carrier can be dissolved in one liter of water at 20°C to form a homogeneous solution.

As used herein the term "water-soluble" with reference to the PTH compound means that at least 1 g of the PTH compound can be dissolved in one liter of water at 20°C to form a homogeneous solution. Accordingly, the term "water-insoluble" with reference to the PTH compound means that less than 1 g of the PTH compound can be dissolved in one liter of water at 20°C to form a homogeneous solution.

As used herein, the term "hydrogel" means a hydrophilic or amphiphilic polymeric network composed of homopolymers or copolymers, which is insoluble due to the presence of covalent chemical crosslinks. The crosslinks provide the network structure and physical integrity.

As used herein the term "thermogelling" means a compound that is a liquid or a low viscosity solution having a viscosity of less than 500 cps at 25°C at a shear rate of about 0.1 /second at a low temperature, which low temperature ranges between about 0°C to about 10°C, but which is a higher viscosity compound of less than 10000 cps at 25°C at a shear rate of about 0.1/second at a higher temperature, which higher temperature ranges between about 30°C to about 40°C, such as at about 37°C.

As used herein, the term "PEG-based" in relation to a moiety or reagent means that said moiety or reagent comprises PEG. Preferably, a PEG-based moiety or reagent comprises at least 10% (w/w) PEG, such as at least 20% (w/w) PEG, such as at least 30% (w/w) PEG, such as at least 40% (w/w) PEG, such as at least 50% (w/w), such as at least 60 (w/w) PEG, such as at least 70% (w/w) PEG, such as at least 80% (w/w) PEG, such as at least 90% (w/w) PEG, such as at least 95%. The remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 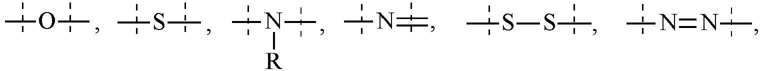 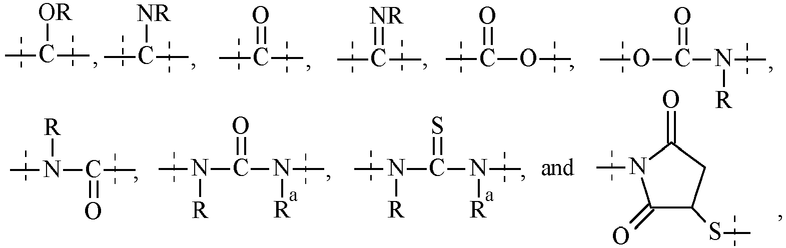 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

As used herein, the term "PEG-based comprising at least X% PEG" in relation to a moiety or reagent means that said moiety or reagent comprises at least X% (w/w) ethylene glycol units (-CH₂CH₂O-), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 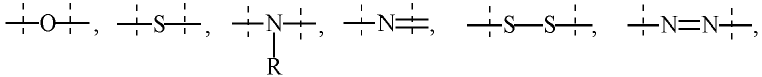 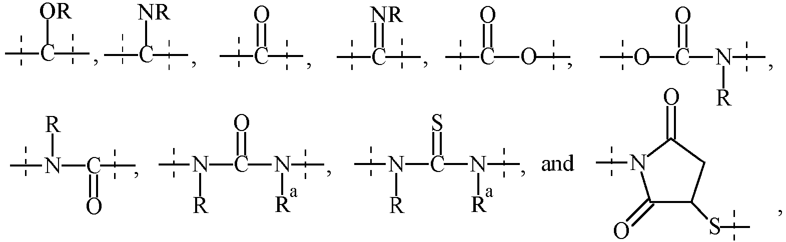 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The term "hyaluronic acid-based comprising at least X% hyaluronic acid" is used accordingly.

The term "spacer moiety" as used here in means any moiety that connects two other moieties. Preferably, a spacer moiety is selected from the group consisting of -T-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
each T is independently selected from the group consisting of phenyl, naphthyl, indenyl, indanyl, tetralinyl, C₃₋₁₀ cycloalkyl, 3- to 10-membered heterocyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.

The term "substituted" as used herein means that one or more -H atom(s) of a molecule or moiety are replaced by a different atom or a group of atoms, which are referred to as "substituent".

Preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(oR^{x3})(R^{x3a})-, -N(R^{x3})C(O)N(R^{x3a})-, and -OC(O)N(R^{x3})-;
-R^{x1}, -R^{x1a}, -R^{x1b} are independently of each other selected from the group consisting of -H, -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-; -S(O)₂-, -S(O)-, -N(R^{x3})S(O)₂N(R^{x3a})-, -S-, -N(R^{x3})-, -OC(oR^{x3})(R^{x3a})-, -N(R^{x3})C(O)N(R^{x3a})-, and -OC(O)N(R^{x3})-;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(0)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x3}, -R^{x3a}, -R^{x4}, -R^{x4a}, -R^{x4b} is independently selected from the group consisting of -H and C₁₋₆ alkyl; wherein C₁₋₆ alkyl is optionally substituted with one or more halogen, which are the same or different.

More preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₁₀ alkyl, C₂₋₁₀ alkenyl, and C₂₋₁₀ alkynyl; wherein -T⁰, C₁₋₁₀ alkyl, C₂₋₁₀ alkenyl, and C₂₋₁₀ alkynyl are optionally substituted with one or more -R^{x2}, which are the same or different and wherein C₁₋₁₀ alkyl, C₂₋₁₀ alkenyl, and C₂₋₁₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(oR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x4}, -R^{x4a}, -R^{x4b} is independently selected from the group consisting of -H, halogen, C₁₋₆ alkyl, C₂₋₆ alkenyl, and C₂₋₆ alkynyl;

Even more preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₆ alkyl, C₂₋₆ alkenyl, and C₂₋₆ alkynyl; wherein -T⁰, C₁₋₆ alkyl, C₂₋₆ alkenyl, and C₂₋₆ alkynyl are optionally substituted with one or more -R^{x2}, which are the same or different and wherein C₁₋₆ alkyl, C₂₋₆ alkenyl, and C₂₋₆ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(oR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x2}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different.

Preferably, a maximum of 6 -H atoms of an optionally substituted molecule are independently replaced by a substituent, e.g. 5 -H atoms are independently replaced by a substituent, 4 -H atoms are independently replaced by a substituent, 3 -H atoms are independently replaced by a substituent, 2 -H atoms are independently replaced by a substituent, or 1 -H atom is replaced by a substituent.

The term "interrupted" means that a moiety is inserted between two carbon atoms or - if the insertion is at one of the moiety's ends - between a carbon or heteroatom and a hydrogen atom, preferably between a carbon and a hydrogen atom.

As used herein, the term "C₁₋₄ alkyl" alone or in combination means a straight-chain or branched alkyl moiety having 1 to 4 carbon atoms. If present at the end of a molecule, examples of straight-chain or branched C₁₋₄ alkyl are methyl, ethyl, n-propyl, isopropyl, n-butyl, isobutyl, sec-butyl and tert-butyl. When two moieties of a molecule are linked by the C₁₋₄ alkyl, then examples for such C₁₋₄ alkyl groups are -CH₂-, -CH₂-CH₂-, -CH(CH₃)-, -CH₂-CH₂-CH₂-, -CH(C₂H₅)-, -C(CH₃)₂-. Each hydrogen of a C₁₋₄ alkyl carbon may optionally be replaced by a substituent as defined above. Optionally, a C₁₋₄ alkyl may be interrupted by one or more moieties as defined below.

As used herein, the term "C₁₋₆ alkyl" alone or in combination means a straight-chain or branched alkyl moiety having 1 to 6 carbon atoms. If present at the end of a molecule, examples of straight-chain and branched C₁₋₆ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the C₁₋₆ alkyl group, then examples for such C₁₋₆ alkyl groups are -CH₂-, -CH₂-CH₂-, -CH(CH₃)-, -CH₂-CH₂-CH₂-, -CH(C₂H₅)- and -C(CH₃)₂-. Each hydrogen atom of a C₁₋₆ carbon may optionally be replaced by a substituent as defined above. Optionally, a C₁₋₆ alkyl may be interrupted by one or more moieties as defined below.

Accordingly, "C₁₋₁₀ alkyl", "C₁₋₂₀ alkyl" or "C₁₋₅₀ alkyl" means an alkyl chain having 1 to 10, 1 to 20 or 1 to 50 carbon atoms, respectively, wherein each hydrogen atom of the C₁₋₁₀, C₁₋₂₀ or C₁₋₅₀ carbon may optionally be replaced by a substituent as defined above. Optionally, a C₁₋₁₀ or C₁₋₅₀ alkyl may be interrupted by one or more moieties as defined below.

As used herein, the term "C₂₋₆ alkenyl" alone or in combination means a straight-chain or branched hydrocarbon moiety comprising at least one carbon-carbon double bond having 2 to 6 carbon atoms. If present at the end of a molecule, examples are -CH=CH₂, -CH=CH-CH₃, -CH₂-CH=CH₂, -CH=CHCH₂-CH₃ and -CH=CH-CH=CH₂. When two moieties of a molecule are linked by the C₂₋₆ alkenyl group, then an example for such C₂₋₆ alkenyl is -CH=CH-. Each hydrogen atom of a C₂₋₆ alkenyl moiety may optionally be replaced by a substituent as defined above. Optionally, a C₂₋₆ alkenyl may be interrupted by one or more moieties as defined below.

Accordingly, the term "C₂₋₁₀ alkenyl", "C₂₋₂₀ alkenyl" or "C₂₋₅₀ alkenyl" alone or in combination means a straight-chain or branched hydrocarbon moiety comprising at least one carbon-carbon double bond having 2 to 10, 2 to 20 or 2 to 50 carbon atoms. Each hydrogen atom of a C₂₋₁₀ alkenyl, C₂₋₂₀ alkenyl or C₂₋₅₀ alkenyl group may optionally be replaced by a substituent as defined above. Optionally, a C₂₋₁₀ alkenyl, C₂₋₂₀ alkenyl or C₂₋₅₀ alkenyl may be interrupted by one or more moieties as defined below.

As used herein, the term "C₂₋₆ alkynyl" alone or in combination means straight-chain or branched hydrocarbon moiety comprising at least one carbon-carbon triple bond having 2 to 6 carbon atoms. If present at the end of a molecule, examples are -C≡CH, -CH₂-C≡CH, CH₂-CH₂-C≡CH and CH₂-C≡C-CH₃. When two moieties of a molecule are linked by the alkynyl group, then an example is -C≡C-. Each hydrogen atom of a C₂₋₆ alkynyl group may optionally be replaced by a substituent as defined above. Optionally, one or more double bond(s) may occur. Optionally, a C₂₋₆ alkynyl may be interrupted by one or more moieties as defined below.

Accordingly, as used herein, the term "C₂₋₁₀ alkynyl", "C₂₋₂₀ alkynyl" and "C₂₋₅₀ alkynyl" alone or in combination means a straight-chain or branched hydrocarbon moiety comprising at least one carbon-carbon triple bond having 2 to 10, 2 to 20 or 2 to 50 carbon atoms, respectively. Each hydrogen atom of a C₂₋₁₀ alkynyl, C₂₋₂₀ alkynyl or C₂₋₅₀ alkynyl group may optionally be replaced by a substituent as defined above. Optionally, one or more double bond(s) may occur. Optionally, a C₂₋₁₀ alkynyl, C₂₋₂₀ alkynyl or C₂₋₅₀ alkynyl may be interrupted by one or more moieties as defined below.

As mentioned above, a C₁₋₄ alkyl, C₁₋₆ alkyl, C₁₋₁₀ alkyl, C₁₋₂₀ alkyl, C₁₋₅₀ alkyl, C₂₋₆ alkenyl, C₂₋₁₀ alkenyl, C₂₋₂₀ alkenyl, C₂₋₅₀ alkenyl, C₂₋₆ alkynyl, C₂₋₁₀ alkynyl, C₂₋₂₀ alkenyl or C₂₋₅₀ alkynyl may optionally be interrupted by one or more moieties which are preferably selected from the group consisting of 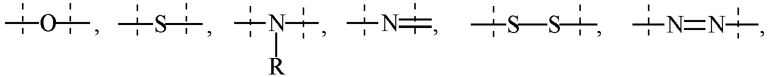 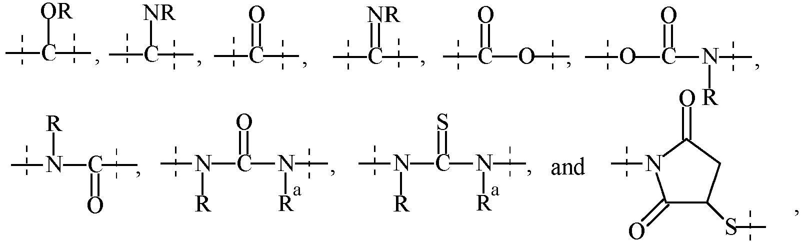 wherein
dashed lines indicate attachment to the remainder of the moiety or reagent; and
-R and -R^{a} are independently of each other selected from the group consisting of -H, methyl, ethyl, propyl, butyl, pentyl and hexyl.

As used herein, the term "C₃₋₁₀ cycloalkyl" means a cyclic alkyl chain having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl or cyclodecyl. Each hydrogen atom of a C₃₋₁₀ cycloalkyl carbon may be replaced by a substituent as defined above. The term "C₃₋₁₀ cycloalkyl" also includes bridged bicycles like norbornane or norbornene.

The term "8- to 30-membered carbopolycyclyl" or "8- to 30-membered carbopolycycle" means a cyclic moiety of two or more rings with 8 to 30 ring atoms, where two neighboring rings share at least one ring atom and that may contain up to the maximum number of double bonds (aromatic or non-aromatic ring which is fully, partially or un-saturated). Preferably a 8- to 30-membered carbopolycyclyl means a cyclic moiety of two, three, four or five rings, more preferably of two, three or four rings.

As used herein, the term "3- to 10-membered heterocyclyl" or "3- to 10-membered heterocycle" means a ring with 3, 4, 5, 6, 7, 8, 9 or 10 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for 3- to 10-membered heterocycles include but are not limited to aziridine, oxirane, thiirane, azirine, oxirene, thiirene, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 3- to 10-membered heterocyclyl or 3- to 10-membered heterocyclic group may be replaced by a substituent as defined below.

As used herein, the term "8- to 11-membered heterobicyclyl" or "8- to 11-membered heterobicycle" means a heterocyclic moiety of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for an 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8- to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

Similary, the term "8- to 30-membered heteropolycyclyl" or "8- to 30-membered heteropolycycle" means a heterocyclic moiety of more than two rings with 8 to 30 ring atoms, preferably of three, four or five rings, where two neighboring rings share at least one ring atom and that may contain up to the maximum number of double bonds (aromatic or non-aromatic ring which is fully, partially or unsaturated), wherein at least one ring atom up to 10 ring atoms are replaced by a heteroatom selected from the group of sulfur (including -S(O)-, -S(O)₂-), oxygen and nitrogen (including =N(O)-) and wherein the ring is linked to the rest of a molecule via a carbon or nitrogen atom.

It is understood that the phrase "the pair R^{x}/R^{y} is joined together with the atom to which they are attached to form a C₃₋₁₀ cycloalkyl or a 3- to 10-membered heterocyclyl" in relation with a moiety of the structure 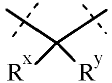
means that R^{x} and R^{y} form the following structure: 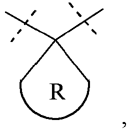
wherein R is C₃₋₁₀ cycloalkyl or 3- to 10-membered heterocyclyl.

It is also understood that the phrase "the pair R^{x}/R^{y} is joint together with the atoms to which they are attached to form a ring A" in relation with a moiety of the structure 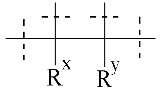 means that R^{x} and R^{y} form the following structure:

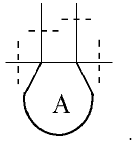

As used herein, "halogen" means fluoro, chloro, bromo or iodo. It is generally preferred that halogen is fluoro or chloro.

In general, the term "comprise" or "comprising" also encompasses "consist of" or "consisting of'.

Administration of the PTH compound is preferably via subcutaneous administration. In another embodiment administration of the PTH compound is via intravenous administration. In a further embodiment administration of the PTH compound is via intramuscular administration.

The time period between two consecutive administrations, preferably between two consecutive subcutaneous administrations, i.e. the administration interval, is preferably at least 24 hours, such as 24 hours, 36 hours, 48 hours, 60 hours, 72 hours, every 84 hours, 96 hours, 108 hours, 120 hours, 132 hours, 144 hours, 156 hours, one week, two weeks, three weeks or four weeks.

**In** one embodiment the time period between two consecutive administrations, preferably between two consecutive subcutaneous administrations, is 24 hours.

**In** another embodiment the time period between two consecutive administrations, preferably between two consecutive subcutaneous administrations, is 48 hours.

**In** another embodiment the time period between two consecutive administrations, preferably between two consecutive subcutaneous administrations, is 72 hours.

**In** another embodiment the time period between two consecutive administrations, preferably between two consecutive subcutaneous administrations, is 96 hours.

**In** another embodiment the time period between two consecutive administrations, preferably between two consecutive subcutaneous administrations, is 120 hours.

**In** another embodiment the time period between two consecutive administrations, preferably between two consecutive subcutaneous administrations, is 144 hours.

**In** another embodiment the time period between two consecutive administrations, preferably between two consecutive subcutaneous administrations, is one week.

The peak to trough ratio measured in each administration interval is less than 4, preferably less than 3.8, more preferably less than 3.6, even more preferably less than 3.4, even more preferably less than 3.2, even more preferably less than 3, even more preferably less than 2.8, even more preferably less than 2.6, even more preferably less than 2.4, even more preferably less than 2.2 and most preferably less than 2.

Preferably the subcutaneous administration is via subcutaneous injection. Preferably, said subcutaneous injection occurs with a pen device.

The PTH compound is a compound of formula (Ia) or a pharmaceutically acceptable salt thereof 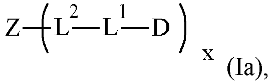 wherein
- D is a PTH moiety;
   - L¹- is of formula (IIb-iii') 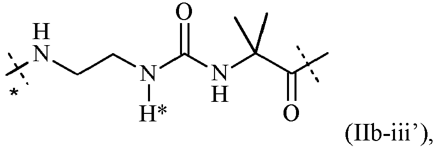 wherein
      the dashed line indicates the attachment to the N-terminal amine functional group of -D through an amide bond;
      the dashed line marked with the asterisk indicates attachment of -L²-Z; and
      wherein -L¹- is optionally substituted, provided that the hydrogen marked with the asterisk in formula (IIb-iii') is not replaced by a substituent;
- L²- is a single chemical bond or a spacer moiety;
- Z is a water-soluble branched PEG-based carrier moiety with a molecular weight ranging from and including 15 kDa to 80 kDa;
   x is 1; and
   wherein the PTH compound has a residual PTH activity compared to the PTH activity of the corresponding free PTH of less than 1%.

It is understood that the compounds of formula (Ia) are PTH prodrugs, more specifically water-soluble PTH prodrugs.

Preferably, -D has the sequence of SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:107, SEQ ID NO:108, SEQ ID NO:109, SEQ ID NO:110, SEQ ID NO:111, SEQ ID NO:112, SEQ ID NO:113, SEQ ID NO:114 or SEQ ID NO:115. More preferably -D has the sequence of SEQ ID NO:50, SEQ ID NO:51, SEQ ID NO:52, SEQ ID NO:110, SEQ ID NO:111 or SEQ ID NO:112.

In one embodiment -D has the sequence of SEQ ID NO:50.

In another embodiment -D has the sequence of SEQ ID NO:52.

In another embodiment -D has the sequence of SEQ ID NO:110.

In another embodiment -D has the sequence of SEQ ID NO:111.

In another embodiment -D has the sequence of SEQ ID NO:112.

Most preferably -D has the sequence of SEQ ID NO:51.

The moiety -L¹- is a reversible prodrug linker from which the drug, i.e. PTH, is released in its free form, i.e. it is a traceless prodrug linker.

It is understood that the nitrogen adjacent to the dashed line marked with the asterisk in formula (IIb-iii') is attached to -L²- through an sp³-hybridized carbon atom.

Preferably the moiety -L¹- of formula (IIb-iii') is not substituted.

In the prodrugs of the present invention -L²- is a chemical bond or a spacer moiety.

In one embodiment -L²- is a chemical bond.

In another embodiment -L²- is a spacer moiety.

When -L²- is other than a single chemical bond, -L²- is preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a}),- -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.

When -L²- is other than a single chemical bond, -L²- is even more preferably selected from -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₁₀ alkyl, C₂₋₁₀ alkenyl, and C₂₋₁₀ alkynyl; wherein -T, C₁₋₁₀ alkyl, C₂₋₁₀ alkenyl, and C₂₋₁₀ alkynyl are optionally substituted with one or more -R^{y2}, which are the same or different, and wherein C₁₋₁₀ alkyl, C₂₋₁₀ alkenyl, and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5}) C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

When -L²- is other than a single chemical bond, -L²- is even more preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently selected from the group consisting of -H, -T, C₁₋₁₀ alkyl, C₂₋₁₀ alkenyl, and C₂₋₁₀ alkynyl;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl;
each -R^{y2} is independently selected from the group consisting of halogen, and C₁₋₆ alkyl; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

Even more preferably, -L²- is a C₁₋₂₀ alkyl chain, which is optionally interrupted by one or more groups independently selected from -O-, -T- and -C(O)N(R^{y1})-; and which C₁₋₂₀ alkyl chain is optionally substituted with one or more groups independently selected from -OH, -T and -C(O)N(R^{y6}R^{y6a}); wherein -R^{y1}, -R^{y6}, -R^{y6a} are independently selected from the group consisting of H and C₁₋₄ alkyl and wherein T is selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl.

Preferably, -L²- has a molecular weight in the range of from 14 g/mol to 750 g/mol.

Preferably, -L²- comprises a moiety selected from 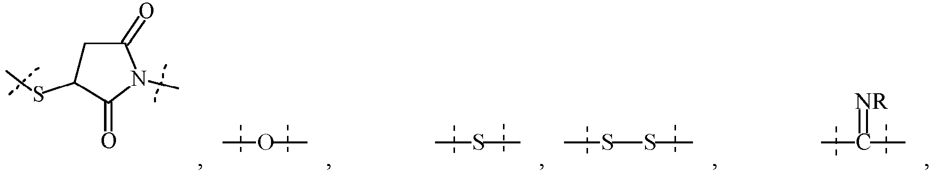 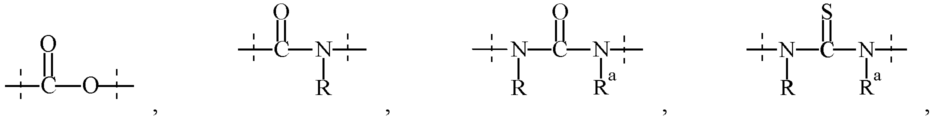 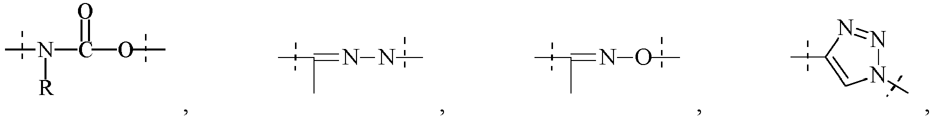 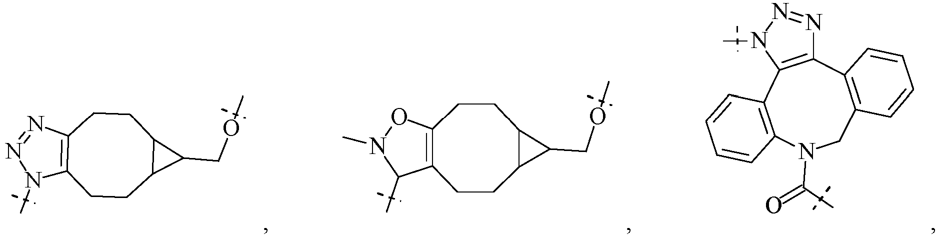 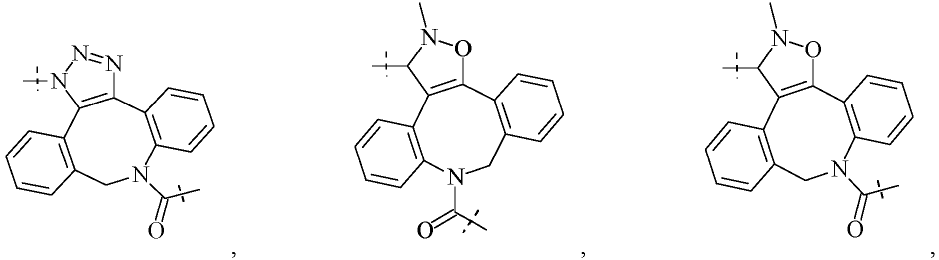 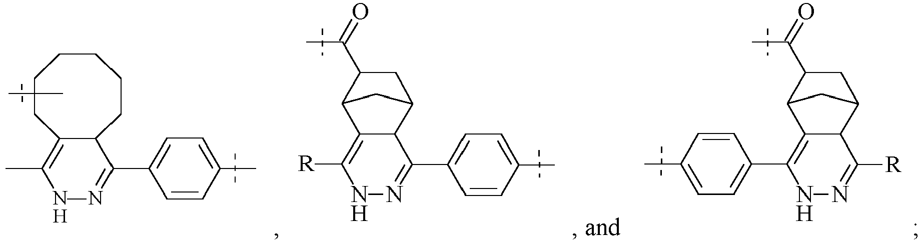 wherein
dashed lines indicate attachment to the rest of -L²-, -L¹- and/or -Z, respectively; and
-R and -R^{a} are independently of each other selected from the group consisting of -H, methyl, ethyl, propyl, butyl, pentyl and hexyl.

In one preferred embodiment -L²- has a chain length of 1 to 20 atoms.

As used herein the term "chain length" with regard to the moiety -L²- refers to the number of atoms of -L²- present in the shortest connection between -L¹- and -Z.

Preferably, -L²- is of formula (i) 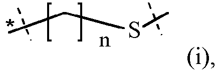 wherein
the dashed line marked with the asterisk indicates attachment to -L¹-;
the unmarked dashed line indicates attachment to -Z;
n is selected from the group consisting of 0, 1, 2, 3, 4, 5, 6, 7, 8, 9, 10, 11, 12, 13, 14, 15, 16, 17 and 18; and
wherein the moiety of formula (i) is optionally substituted.

Preferably, n of formula (i) is selected from the group consisting of 3, 4, 5, 6, 7, 8, and 9. Even more preferably n of formula (i) is 4, 5, 6, or 7. In one embodiment n of formula (i) is 4. In another embodiment n of formula (i) is 5. In another embodiment n of formula (i) is 6.

In another preferred embodiment the moiety -L¹-L²- is selected from the group consisting of 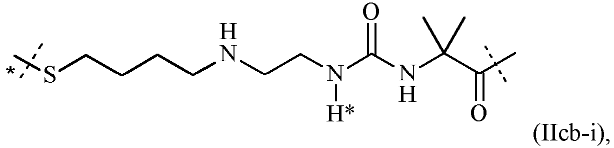 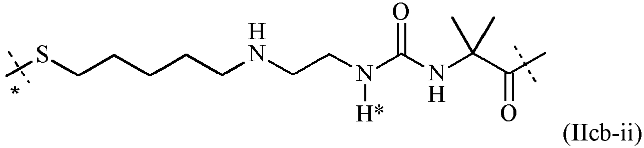 and 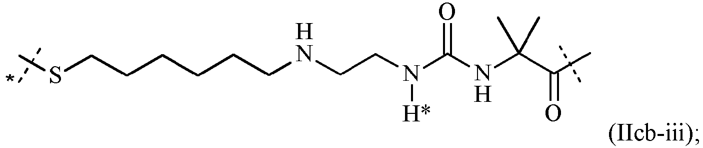 wherein
the unmarked dashed line indicates the attachment to the N-terminal amine functional group of -D which is a PTH moiety by forming an amide bond; and
the dashed line marked with the asterisk indicates attachment to -Z.

In a preferred embodiment the moiety -L¹-L²- is of formula (IIca-ii).

In another preferred embodiment the moiety -L¹-L²- is of formula (IIcb-iii).

-Z is a branched PEG-based polymer. In one embodiment -Z is a branched PEG-based polymer having one, two, three, four, five or six branching points. Preferably, -Z is a branched PEG-based polymer having one, two or three branching points. In one embodiment -Z is a branched PEG-based polymer having one branching point. In another embodiment -Z is a branched PEG-based polymer having two branching points. In another embodiment -Z is a branched PEG-based polymer having three branching points.

A branching point is preferably selected from the group consisting of -N<, -CH< and >C<.

-Z has a molecular weight ranging from and including 15 kDa to 80 kDa.

In one embodiment the molecular weight of such branched moiety -Z is about 20 kDa. In another embodiment the molecular weight of such branched moiety -Z is about 30 kDa. In another embodiment the molecular weight of such a branched moiety -Z is about 40 kDa. In another embodiment the molecular weight of such a branched moiety -Z is about 50 kDa. In another embodiment the molecular weight of such a branched moiety -Z is about 60 kDa. In another embodiment the molecular weight of such a branched moiety -Z is about 70 kDa. Most preferably, such branched moiety -Z has a molecular weight of about 40 kDa.

Preferably, -Z comprises a moiety

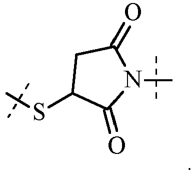

In an equally preferred embodiment -Z comprises an amide bond.

Preferably -Z comprises a moiety of formula (a) 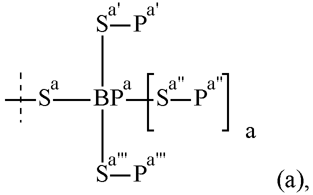 wherein
the dashed line indicates attachment to -L²- or to the remainder of -Z;
BP^{a} is a branching point selected from the group consisting of -N<, -CR< and >C<;
-R is selected from the group consisting of -H and C₁₋₆ alkyl;
a is 0 if BP^{a} is -N< or -CR< and n is 1 if BP^{a} is >C<;
-S^{a}-, -S^{a'}-, -S^{a"}- and -S^{a‴}- are independently of each other a chemical bond or are selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different; and
-P^{a'}, -P^{a"} and -P^{a‴} are independently a polymeric moiety.

In one embodiment BP^{a} of formula (a) is -N<.

In another embodiment BP^{a} of formula (a) is >C<.

In a preferred embodiment BP^{a} of formula (a) is -CR<. Preferably, -R is -H. Accordingly, a of formula (a) is 0.

In one embodiment -S^{a}- of formula (a) is a chemical bond.

In another embodiment -S^{a}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -T-, -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -T- is a 3- to 10-membered heterocyclyl; and -R⁴ and -R^{4a} are independently selected from the group consisting of -H, methyl, ethyl, propyl and butyl.

Preferably -S^{a}- of formula (a) is selected from the group consisting of C₁₋₁₀ alkyl which is interrupted by one or more chemical groups selected from the group consisting of -T-, -C(O)N(R⁴)- and -O-.

In one embodiment -S^{a'}- of formula (a) is a chemical bond.

In another embodiment -S^{a'}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a'}- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a"}- of formula (a) is a chemical bond.

In another embodiment -S^{a"}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a"}- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a‴}- of formula (a) is a chemical bond.

In another embodiment -S^{a‴}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a‴}- of formula (a) is selected from the group consisting of methyl, ethyl, propyl, butyl, which are optionally interrupted by one or more chemical groups selected from the group consisting of -O-, -C(O)- and -C(O)N(R⁴)-.

Preferably, -P^{a'}, -P^{a"} and -P^{a‴} of formula (a) independently comprise a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

More preferably, -P^{a'}, -P^{a"} and -P^{a‴} of formula (a) independently comprise a PEG-based moiety. Even more preferably, -P^{a'}, -P^{a"} and -P^{a‴} of formula (a) independently comprise a PEG-based moiety comprising at least 20% PEG, even more preferably at least 30%, even more preferably at least 40% PEG, even more preferably at least 50% PEG, even more preferably at least 60% PEG, even more preferably at least 70% PEG, even more preferably at least 80% PEG and most preferably at least 90% PEG.

Preferably, -P^{a'}, -P^{a"} and -P^{a‴} of formula (a) independently have a molecular weight ranging from and including 5 kDa to 50 kDa, more preferably have a molecular weight ranging from and including 5 kDa to 40 kDa, even more preferably ranging from and including 7.5 kDa to 35 kDa, even more preferably ranging from and 7.5 to 30 kDa, even more preferably ranging from and including 10 to 30 kDa.

In one embodiment -P^{a'}, -P^{a"} and -P^{a‴} of formula (a) have a molecular weight of about 5 kDa.

In another embodiment -P^{a'}, -P^{a"} and -P^{a‴} of formula (a) have a molecular weight of about 7.5 kDa.

In another embodiment -P^{a'}, -P^{a"} and -P^{a‴} of formula (a) have a molecular weight of about 10 kDa.

In another embodiment -P^{a'}, -P^{a"} and -P^{a‴} of formula (a) have a molecular weight of about 12.5 kDa.

In another embodiment -P^{a'}, -P^{a"} and -P^{a‴} of formula (a) have a molecular weight of about 15 kDa.

In another embodiment -P^{a'}, -P^{a"} and -P^{a‴} of formula (a) have a molecular weight of about 20 kDa.

In one embodiment -Z comprises one moiety of formula (a).

In another embodiment -Z comprises two moieties of formula (a).

In another embodiment -Z comprises three moieties of formula (a).

Preferably, -Z is a moiety of formula (a).

More preferably, -Z comprises a moiety of formula (b) 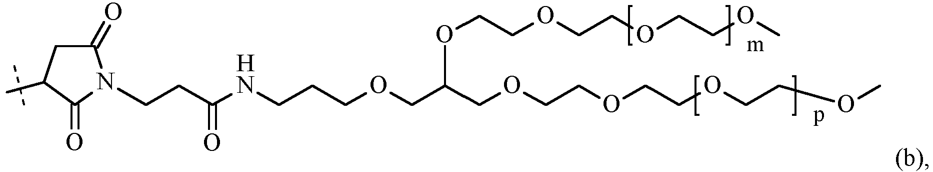 wherein
the dashed line indicates attachment to -L²- or to the remainder of -Z; and
m and p are independently of each other an integer ranging from and including 400 to 500.

Preferably, m and p of formula (b) are the same integer.

Most preferably m and p of formula (b) are about 450.

Preferably, -Z is a moiety of formula (b).

In a preferred embodiment the PTH prodrug of the present invention is of formula (IIf-i): 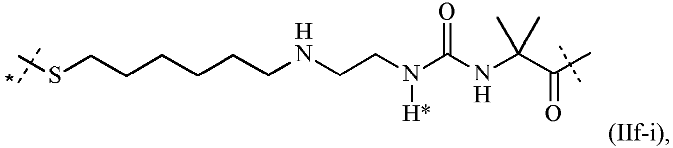 wherein
the unmarked dashed line indicates the attachment to the N-terminal amine functional group of -D which is a PTH moiety by forming an amide bond; and
the dashed line marked with the asterisk indicates attachment to a moiety 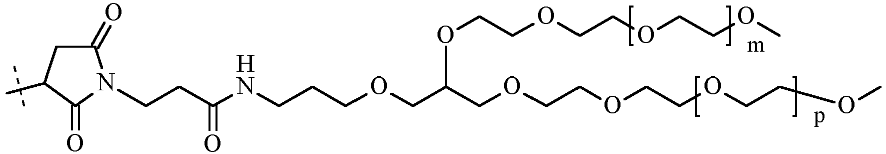 wherein
   m and p are independently an integer ranging from and including 400 to 500.

The residual activity of the PTH prodrug is less than 1%, preferably less than 0.1%, more preferably less than 0.01%, even more preferably less than 0.001% and most preferably less than 0.0001%.

As used herein the term "residual activity" refers to the activity exhibited by the PTH prodrug with the PTH moiety bound to a carrier in relation to the activity exhibited by the corresponding free PTH. In this context the term "activity" refers to binding to an activation domain of the PTH/PTHrP1 receptor resulting in activation of adenylate cyclase to generate cAMP, phospholipase C to generate intracellular calcium, or osteoblastic expression of RANKL (which binds to RANK (Receptor Activator of Nuclear Factor kB) on osteoclasts. It is understood that measuring the residual activity of the PTH prodrug of the present invention takes time during which a certain amount of PTH may be released from the PTH prodrug of the present invention and that such released PTH will distort the results measured for the PTH prodrug. It is thus accepted practice to test the residual activity of a prodrug with a conjugate in which the drug moiety, in this case PTH, is non-reversibly, i.e. stably, bound to a carrier, which as closely as possible resembles the structure of the PTH prodrug for which residual activity is to be measured.

Preferably, the pharmaceutical composition of the present invention has a pH ranging from and including pH 3 to pH 8. More preferably, the pharmaceutical composition has a pH ranging from and including pH 4 to pH 6. Most preferably, the pharmaceutical composition has a pH ranging from and including pH 4 to pH 5.

In one embodiment the pharmaceutical composition of the present invention is a liquid or suspension formulation. It is understood that the pharmaceutical composition is a suspension formulation if the controlled-release PTH compound of the present invention is water-insoluble.

In another embodiment the pharmaceutical composition of the present invention is a dry formulation which is reconstituted before administration to a patient.

Such liquid, suspension, dry or reconstituted pharmaceutical composition comprises at least one excipient. Excipients used in parenteral formulations may be categorized as, for example, buffering agents, isotonicity modifiers, preservatives, stabilizers, anti-adsorption agents, oxidation protection agents, viscosifiers/viscosity enhancing agents, or other auxiliary agents. However, in some cases, one excipient may have dual or triple functions. Preferably, the at least one excipient comprised in the pharmaceutical composition of the present invention is selected from the group consisting of
(i) Buffering agents: physiologically tolerated buffers to maintain pH in a desired range, such as sodium phosphate, bicarbonate, succinate, histidine, citrate and acetate, sulphate, nitrate, chloride, pyruvate; antacids such as Mg(OH)₂ or ZnCO₃ may be also used;
(ii) Isotonicity modifiers: to minimize pain that can result from cell damage due to osmotic pressure differences at the injection depot; glycerin and sodium chloride are examples; effective concentrations can be determined by osmometry using an assumed osmolality of 285-315 mOsmol/kg for serum;
(iii) Preservatives and/or antimicrobials: multidose parenteral formulations require the addition of preservatives at a sufficient concentration to minimize risk of patients becoming infected upon injection and corresponding regulatory requirements have been established; typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride;
(iv) Stabilizers: Stabilisation is achieved by strengthening of the protein-stabilising forces, by destabilisation of the denatured state, or by direct binding of excipients to the protein; stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives; in addition, oligomers or polymers such as cyclodextrins, dextran, dendrimers, PEG or PVP or protamine or HSA may be used;
(v) Anti-adsorption agents: Mainly ionic or non-ionic surfactants or other proteins or soluble polymers are used to coat or adsorb competitively to the inner surface of the formulation's container; e.g., poloxamer (Pluronic F-68), PEG dodecyl ether (Brij 35), polysorbate 20 and 80, dextran, polyethylene glycol, PEG-polyhistidine, BSA and HSA and gelatins; chosen concentration and type of excipient depends on the effect to be avoided but typically a monolayer of surfactant is formed at the interface just above the CMC value;
(vi) Oxidation protection agents: antioxidants such as ascorbic acid, ectoine, methionine, glutathione, monothioglycerol, morin, polyethylenimine (PEI), propyl gallate, and vitamin E; chelating agents such as citric acid, EDTA, hexaphosphate, and thioglycolic acid may also be used;
(vii) Viscosifiers or viscosity enhancers: in case of a suspension retard settling of the particles in the vial and syringe and are used in order to facilitate mixing and resuspension of the particles and to make the suspension easier to inject (i.e., low force on the syringe plunger); suitable viscosifiers or viscosity enhancers are, for example, carbomer viscosifiers like Carbopol 940, Carbopol Ultrez 10, cellulose derivatives like hydroxypropylmethylcellulose (hypromellose, HPMC) or diethylaminoethyl cellulose (DEAE or DEAE-C), colloidal magnesium silicate (Veegum) or sodium silicate, hydroxyapatite gel, tricalcium phosphate gel, xanthans, carrageenans like Satia gum UTC 30, aliphatic poly(hydroxy acids), such as poly(D,L- or L-lactic acid) (PLA) and poly(glycolic acid) (PGA) and their copolymers (PLGA), terpolymers of D,L-lactide, glycolide and caprolactone, poloxamers, hydrophilic poly(oxyethylene) blocks and hydrophobic poly(oxypropylene) blocks to make up a triblock of poly(oxyethylene)-poly(oxypropylene)-poly(oxyethylene) (e.g. Pluronic^{®}), polyetherester copolymer, such as a polyethylene glycol terephthalate/polybutylene terephthalate copolymer, sucrose acetate isobutyrate (SAIB), dextran or derivatives thereof, combinations of dextrans and PEG, polydimethylsiloxane, collagen, chitosan, polyvinyl alcohol (PVA) and derivatives, polyalkylimides, poly (acrylamide-co-diallyldimethyl ammonium (DADMA)), polyvinylpyrrolidone (PVP), glycosaminoglycans (GAGs) such as dermatan sulfate, chondroitin sulfate, keratan sulfate, heparin, heparan sulfate, hyaluronan, ABA triblock or AB block copolymers composed of hydrophobic A-blocks, such as polylactide (PLA) or poly(lactide-co-glycolide) (PLGA), and hydrophilic B-blocks, such as polyethylene glycol (PEG) or polyvinyl pyrrolidone; such block copolymers as well as the abovementioned poloxamers may exhibit reverse thermal gelation behavior (fluid state at room temperature to facilitate administration and gel state above sol-gel transition temperature at body temperature after injection);
(viii) Spreading or diffusing agent: modifies the permeability of connective tissue through the hydrolysis of components of the extracellular matrix in the intrastitial space such as hyaluronic acid, a polysaccharide found in the intercellular space of connective tissue; a spreading agent such as hyaluronidase temporarily decreases the viscosity of the extracellular matrix and promotes diffusion of injected drugs; and
(ix) Other auxiliary agents: such as wetting agents, viscosity modifiers, antibiotics, hyaluronidase; acids and bases such as hydrochloric acid and sodium hydroxide are auxiliary agents necessary for pH adjustment during manufacture.

### Examples

### Materials and Methods

Side chain protected PTH(1-34) (SEQ ID NO:51) on TCP resin having Boc protected N-terminus and ivDde protected side chain of Lys26 (synthesized by Fmoc-strategy) was obtained from custom peptide synthesis providers.

Side chain protected PTH(1-34) on TCP resin having Fmoc protected N-terminus (synthesized by Fmoc-strategy) was obtained from custom peptide synthesis providers.

PEG 2x20 kDa maleimide, Sunbright GL2-400MA was purchased from NOF Europe N.V., Grobbendonk, Belgium. S-Trityl-6-mercaptohexanoic acid was purchased from Polypeptide, Strasbourg, France. HATU was obtained from Merck Biosciences GmbH, Schwalbach/Ts, Germany. Fmoc-N-Me-Asp(OBn)-OH was obtained from Peptide International Inc., Louisville, KY, USA. Fmoc-Aib-OH was purchased from Iris Biotech GmbH, Marktredwitz, Germany. All other chemicals and reagents were purchased from Sigma Aldrich GmbH, Taufkirchen, Germany, unless a different supplier is mentioned.

Compound **11a** (examples 11-15) was synthesized following the procedure described in patent WO29095479A2, example 1.

Syringes equipped with polyethylenene frits (MultiSynTech GmbH, Witten, Germany) were used as reaction vessels or for washing steps of peptide resins.

General procedure for the removal of ivDde protecting group from side chain protected PTH on resin: The resin was pre-swollen in DMF for 30 min and the solvent was discarded. The ivDde group was removed by incubating the resin with DMF/hydrazine hydrate 4/1 (v/v, 2.5 mL/g resin) for 8 x 15 min. For each step fresh DMF/hydrazine hydrate solution was used. Finally, the resin was washed with DMF (10 x), DCM (10 x) and dried *in vacuo.*

General procedure for the removal of Fmoc protecting group from protected PTH on resin: The resin was pre-swollen in DMF for 30 min and the solvent was discarded. The Fmoc group was removed by incubating the resin with DMF/piperidine/DBU 96/2/2 (v/v/v, 2.5 mL/g resin) for 3 x 10 min. For each step fresh DMF/piperidine/DBU hsolution was used. Finally, the resin was washed with DMF (10 x), DCM (10 x) and dried *in vacuo.*

### RP-HPLC purification:

For preparative RP-HPLC a Waters 600 controller and a 2487 Dual Absorbance Detector was used, equipped with the following columns: Waters XBridge^{™} BEH300 Prep C18 5 µm, 150 x 10 mm, flow rate 6 mL/min, or Waters XBridge^{™} BEH300 Prep C18 10 µm, 150 x 30 mm, flow rate 40 mL/min. Linear gradients of solvent system A (water containing 0.1 % TFA v/v) and solvent system B (acetonitrile containing 0.1 % TFA v/v) were used. HPLC fractions containing product were pooled and lyophilized if not stated otherwise.

### Flash Chromatography:

Flash chromatography purifications were performed on an Isolera One system from Biotage AB, Sweden, using Biotage KP-Sil silica cartridges and n-heptane and ethyl acetate as eluents. Products were detected at 254 nm.

### Ion exchange chromatography:

Ion exchange chromatography (IEX) was performed using an Amersham Bioscience AEKTAbasic system equipped with a MacroCap SP cation exchanger column (Amersham Bioscience/GE Healthcare). 17 mM acetic acid pH 4.5 (solvent A) and 17 mM acetic acid, 1 M NaCl, pH 4.5 (solvent B) were used as mobile phases.

### Size exclusion chromatography:

Size exclusion chromatography (SEC) was performed using an Amersham Bioscience AEKTAbasic system equipped with HiPrep 26/10 desalting columns (Amersham Bioscience/GE Healthcare). 0.1 % (v/v) acetic acid was used as mobile phase.

### Analytical methods

Analytical ultra-performance LC (UPLC)-MS was performed on a Waters Acquity system equipped with a Waters BEH300 C18 column (2.1 x 50 mm, 1.7 µm particle size, flow: 0.25 mL/min; solvent A: water containing 0.04% TFA (v/v), solvent B: acetonitrile containing 0.05% TFA (v/v)) coupled to a LTQ Orbitrap Discovery mass spectrometer from Thermo Scientific or coupled to a Waters Micromass ZQ.

Quantification of plasma total PTH(1-34) concentrations:
Plasma total PTH(1-34) concentrations were determined by quantification of a signature peptide close to the N-terminus (sequence: IQLMHNLGK) and a C-terminal signature peptide (sequence: LQDVHNF) after plasma protein precipitation, followed by sequential digestion with Endoproteinase Lys-C (origin: Lysobacter enzymogenes) and Endoproteinase Glu-C (origin: Staphylococcus aureus V8) of the supernatant. Subsequently, analysis by reversed phase liquid chromatography and detection by mass spectrometry (RP-HPLC-MS) was performed.

Calibration standards of PTH(1-34) conjugate in blank heparin or EDTA plasma were prepared in concentration ranges from 1 to 1000 ng/mL PTH(1-34) eq (dilution with rat plasma) and 1 to 1000 ng/mL PTH(1-34) eq (dilution with monkey plasma).

These solutions were used for the generation of a calibration curve. For quality control, three samples independent from the calibration standard solutions were prepared accordingly. Concentrations at the lower end (3-5 fold concentration of the respective LLOQ), the middle range (0.05 - 0.1 fold concentration of the respective ULOQ) and the upper end (0.5-0.8 fold concentration of the respective ULOQ).

Sample preparation volumes can be altered depending on the targeted signal response after sample preparation. Processing procedure of the protein precipitation is described here for the analysis of plasma samples originated in rat species. Protein precipitation was carried out first by addition of 100 µL of internal standard solution (625 ng/mL of deuterated conjugate) and then by addition of 400 µL of acetonitrile to 50 µL of the plasma sample. 2 times 150 µL of the supernatant were transferred into a new well-plate and evaporated to dryness (under a gentle nitrogen stream at 50 °C). 50 µL of reconstitution solvent (50 mM Tris 0.5 mM CaCl₂ buffer, adjusted to pH 8.0) were used to dissolve the residue. Proteolytic digestion was performed as follows:
20 µg of Lys-C (order number 125-05061, Wako Chemicals GmbH, Neuss, Germany) were dissolved in 80 µL of 10 mM acetic acid. 3 µL of the Lys-C solution were added to each cavity and samples incubated for 15 hours at 37°C. Afterwards 10 µg of Glu-C (order number V1651, Promega GmbH, Mannheim, Germany) were dissolved in 25 µL water, and 1.5 µL of the Glu-C solution added to each cavity and incubation continued for 1.5 hours at 37°C. After incubation samples were acidified with 2 µL water/formic acid 4:6 (v/v) and 10 µL were injected into the UPLC-MS system.

Chromatography was performed on a Waters Acquity BEH300 C18 analytical column (1.7 µm particle size; column dimensions 50 x 2.1 mm). Water (UPLC grade) containing 0.1 % formic acid (v/v) was used as mobile phase A and acetonitrile (UPLC grade) with 0.1 % formic acid as mobile phase B.

Mass analysis was performed on an AB Sciex 6500⁺ QTrap in multiple reaction monitoring (MRM) mode, monitoring the transition m/z 352.0 to 462.1 (signature peptide IQLMHNLGK), 355.4 to 467.1 (signature peptide IQLMHNLGK, internal standard), 436.9 to 631.4 (signature peptide LQDVHNF), and 441.9 to 631.4 (signature peptide LQDVHNF, internal standard).

### Quantification of plasma PEG concentrations:

Plasma total PEG concentrations were determined by quantification of the polymeric part of PTH(1-34) conjugates after plasma protein precipitation and enzymatic digestion of the supernatant. Analysis by size exclusion chromatography and detection by mass spectrometry (SEC-MS) followed.

Calibration standards of PTH(1-34) conjugate in blank heparinized monkey plasma were prepared in concentration ranges from 50 to 4500 ng/mL PEG equivalents.

These solutions were used for the generation of a quadratic calibration curve. Calibration curves were weighted 1/x. For quality control, three samples independent from the calibration standard solutions were prepared accordingly. Concentrations at the lower end (2-4 fold concentration of the LLOQ), the middle range (0.1 - 0.2 fold concentration of the ULOQ) and the upper end (0.8 fold concentration of the ULOQ). Protein precipitation was carried out by addition of 200 µL of precooled (5-10 °C) methanol to 100 µL of the plasma sample. 180 µL of the supernatant were transferred into a new well-plate and evaporated to dryness (under a gentle nitrogen stream at 45 °C). 50 µL of reconstitution solvent (50 mM Tris 0.5 mM CaCl₂ buffer, adjusted to pH 8.0) were used to dissolve the residue. Proteolytic digestion was performed as follows: 20 µg of Lys-C (order number 125-05061, Wako Chemicals GmbH, Neuss, Germany) were dissolved in 80 µL of 10 mM acetic acid. 3 µL of the Lys-C solution were added to each cavity and samples incubated for 15 hours at 37°C. Afterwards 10 µg of Glu-C (order number V1651, Promega GmbH, Mannheim, Germany) were dissolved in 25 µL water, and 1.5 µL of the Glu-C solution added to each cavity and incubation continued for 1.5 hours at 37°C. After incubation samples were acidified with 2 µL water/formic acid 4:6 (v/v) and 5 µL were injected into the SEC-MS system.

SEC-MS analysis was carried out by using an Agilent 1290 UPLC coupled to an Agilent 6460 TripleQuad mass spectrometer via an ESI probe. Acquisition of a distinct precursor ion of the polymer was achieved by applying high voltage in-source fragmentation (200-300V) at the MS interface. Chromatography was performed on a TOSOH TSK Gel SuperAW3000 analytical column (4.0 µm particle size; column dimensions 150 x 6.0 mm) at a flow rate of 0.50 mL/min (T = 65 °C). Water (UPLC grade) containing 0.1 % formic acid (v/v) was used as mobile phase A and acetonitrile (UPLC grade) with 0.1 % formic acid as mobile phase B. The chromatographic setup for sample analysis comprises an isocratic elution of 50% B over 8 minutes.

Mass analysis was performed in single reaction monitoring (SRM) mode, monitoring the transition m/z 133.1 to 45.1.

Quantification of plasma Free PTH concentrations:
Free PTH concentrations in acidified plasma were determined as the sum of peptide PTH(1-34) and peptide PTH(1-33) after plasma protein precipitation, followed by solid phase extraction. Subsequently, analysis using liquid chromatography separation and detection by mass spectrometry (LC-MS) was performed.

Calibration standards of PTH(1-34) and PTH(1-33) in blank acidified EDTA rat plasma were prepared in concentrations ranging from 5.00 to 500 pg/mL in acidified plasma for each analyte. The corresponding concentration range is7.00 to 700 pg/mL for both analytes in neat plasma, as plasma is acidified as volume ratio plasma : 0.5 M citrate buffer pH 4 = 1 : 0.4 v/v.

Standard solutions were used for the generation of a calibration curve. For quality control, three samples were prepared independent of the calibration standard solutions at 15.0, 150 and 400 pg/mL in acidified plasma.

Protein precipitation was carried out by addition of 150 µL of cold acetonitrile to 150 µL of the plasma sample after addition of 50.0 µL of cold internal standard solution, followed by centrifugation. The supernatant was decanted into a new ploypropylene tube and 900 µL of cold water was added. After another centrifugation step, the tubes were kept in ice-water until loading on the SPE column.

Solid phase extraction: the HLB µelution columns were conditioned with 200 µL methanol followed by 200 µL water. The columns were loaded 3 times with 420 µL of the diluted samples by applying positive pressure. The SPE-columns were washed with 200 µL of methanol:water 5:95 v/v. The samples were eluted with 40.0 µL SPE elution solvent (Aceonitrile: water: Trifluoroactic acid 60:40:1 v/v/v), followed by 40.0 µL of water. The elution solvent was left standing on the columns for 2 minutes, after which very gentle pressure was applied for elution.

Separation between metabolites and interfering endogenous compounds was achieved by LC-MS using an Xselect CSH C18 column (2.1x100mm, 2.5 µm) at 50°C and using 0.2% Formic acid and 0.5% dimethyl sulfoxide in water as mobile phase A, 0.5% dimethyl sulfoxide in acetonitrile:methanol (75:25, v/v) as mobile phase B, and operating at a gradient with a flow rate of 0.500 mL/min.

A triple 6500 quadrupole mass spectrometer equipped with a turbo ion spray source was used for detection in positive ion mode. For PTH(1-34), PTH(1-33) and PTH(1-33) (Leu-d₁₀)₃, quantification was done by counting 5 times the same SRM transition.
Quantification is based on multiple reaction monitoring (MRM) of the transitions of m/z: 687.3-787.3 for PTH(1-34)
662.8-757.9 for PTH(1-33)
692.3-793.3 for PTH(1-34) (Leu-d₁₀)₃
667.8-763.9 for PTH(1-33) (Leu-d₁₀)₃

A linear calibration curve with a 1/x² weighing factor was used for both analytes.

Concentrations of free PTH were determined in acidified plasma, as a means to stabilize the analytes. Acidified plasma was prepared by diluting rat EDTA plasma 1.4 times (in case of blank, zero, calibration and QC samples) or rat whole blood (in case of study samples) 1.2 times. Assuming approximately 50% (v/v) of whole blood being plasma, the neat plasma concentrations are approximately 1.4 times higher than the reported concentrations in acidified plasma. Free PTH concentrations are calculated as sum of Free PTH (1-34) and Free PTH (1-33) in PTH(1-34) equivalents).

Due to the reversible nature of the attachment of -L¹- to -D, measurements for PTH receptor activity were made using stable analogs of the PTH prodrugs of the present invention, i.e. they were made using similar structures to those of the PTH prodrugs of the present invention which instead of a reversible attachment of -Z to -D have a stable attachment.

This was necessary, because the PTH prodrugs of the present invention would release PTH in the course of the experiment and said released PTH would have influenced the result.

### Example 1

### Synthesis of linker reagent 1f (not according to the invention)

Linker reagent **1f** was synthesized according to the following scheme:

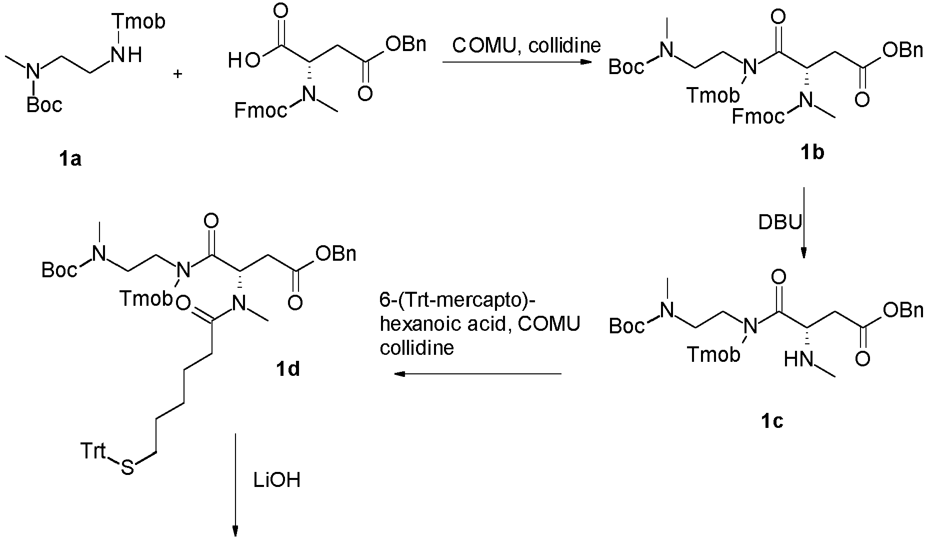

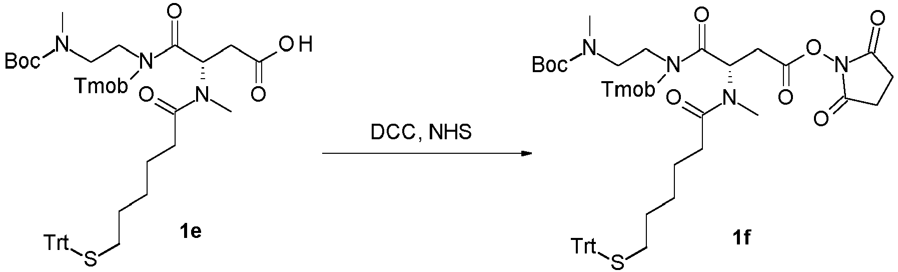

To a solution of N-methyl-N-Boc-ethylenediamine (2 g, 11.48 mmol) and NaCNBH₃ (819 mg, 12.63 mmol) in MeOH (20 mL) was added 2,4,6-trimethoxybenzaldehyde (2.08 g, 10.61 mmol) portion wise. The mixture was stirred at rt for 90 min, acidified with 3 M HCl (4 mL) and stirred further 15 min. The reaction mixture was added to saturated NaHCO₃ solution (200 mL) and extracted 5 x with DCM. The combined organic phases were dried over Na₂SO₄ and the solvents were evaporated *in vacuo.* The resulting N-methyl-N-Boc-N'-Tmob-ethylenediamine **1a** was dried in high vacuum and used in the next reaction step without further purification.

| | |
|---|---|
| Yield: | 3.76 g (11.48 mmol, 89 % purity, **1a**: double Tmob protected product = 8 :1) |
| MS: | m/z 355.22 = [M+H]⁺, (calculated monoisotopic mass = 354.21). |

To a solution of **1a** (2 g, 5.65 mmol) in DCM (24 mL) COMU (4.84 g, 11.3 mmol), N-Fmoc-N-Me-Asp(OBn)-OH (2.08 g, 4.52 mmol) and 2,4,6-collidine (2.65 mL, 20.34 mmol) were added. The reaction mixture was stirred for 3 h at rt, diluted with DCM (250 mL) and washed 3 x with 0.1 M H₂SO₄ (100 mL) and 3 x with brine (100 mL). The aqueous phases were re-extracted with DCM(100 mL). The combined organic phases were dried over Na₂SO₄, filtrated and the residue concentrated to a volume of 24 mL. **1b** was purified using flash chromatography.

| | |
|---|---|
| Yield: | 5.31 g (148 %, 6.66 mmol) |
| MS: | m/z 796.38 = [M+H]⁺, (calculated monoisotopic mass = 795.37). |

To a solution of **1b** (5.31 g, max. 4.52 mmol ref. to N-Fmoc-N-Me-Asp(OBn)-OH) in THF (60 mL) DBU (1.8 mL, 3 % v/v) was added. The solution was stirred for 12 min at rt, diluted with DCM (400 mL) and washed 3 x with 0.1 M H₂SO₄ (150 mL) and 3 x with brine (150 mL). The aqueous phases were re-extracted with DCM (100 mL). The combined organic phases were dried over Na₂SO₄ and filtrated. **1c** was isolated upon evaporation of the solvent and used in the next reaction without further purification.

| | |
|---|---|
| MS: | m/z 574.31 = [M+H]⁺, (calculated monoisotopic mass = 573.30). |

**1c** (5.31 g, 4.52 mmol, crude) was dissolved in acetonitrile (26 mL) and COMU (3.87 g, 9.04 mmol), 6-tritylmercaptohexanoic acid (2.12 g, 5.42 mmol) and 2,4,6-collidine (2.35 mL, 18.08 mmol) were added. The reaction mixture was stirred for 4 h at rt, diluted with DCM (400 mL) and washed 3 x with 0.1 M H₂SO₄ (100 mL) and 3 x with brine (100 mL). The aqueous phases were re-extracted with DCM(100 mL). The combined organic phases were dried over Na₂SO₄, filtered and **1d** was isolated upon evaporation of the solvent. Product **1d** was purified using flash chromatography.

| | |
|---|---|
| Yield: | 2.63 g (62 %, 94 % purity) |
| MS: | m/z 856.41 = [M+H]⁺, (calculated monoisotopic mass = 855.41). |

To a solution of **1d** (2.63 g, 2.78 mmol) in i-PrOH (33 mL) and H₂O (11 mL) was added LiOH (267 mg, 11.12 mmol) and the reaction mixture was stirred for 70 min at rt. The mixture was diluted with DCM (200 mL) and washed 3 x with 0.1 M H₂SO₄ (50 mL) and 3 x with brine (50 mL). The aqueous phases were re-extracted with DCM (100 mL). The combined organic phases were dried over Na₂SO₄, filtered and **1e** was isolated upon evaporation of the solvent. **1e** was purified using flash chromatography.

| | |
|---|---|
| Yield: | 2.1 g (88 %) |
| MS: | m/z 878.4 = [M+Na]⁺, (calculated monoisotopic mass = 837.40). |

To a solution of **1e** (170 mg, 0.198 mmol) in anhydrous DCM (4 mL) were added DCC (123 mg, 0.59 mmol), and a catalytic amount of DMAP. After 5 min, N-hydroxy-succinimide (114 mg, 0.99 mmol) was added and the reaction mixture was stirred at rt for 1 h. The reaction mixture was filtered, the solvent was removed *in vacuo* and the residue was taken up in 90 % acetonitrile plus 0.1 % TFA (3.4 mL). The crude mixture was purified by RP-HPLC. Product fractions were neutralized with 0.5 M pH 7.4 phosphate buffer and concentrated. The remaining aqueous phase was extracted with DCM and **1f** was isolated upon evaporation of the solvent.

| | |
|---|---|
| Yield: | 154 mg (81%) |
| MS: | m/z 953.4 = [M+H]⁺, (calculated monoisotopic mass = 952.43). |

### Example 2

### Synthesis of linker reagent 2g

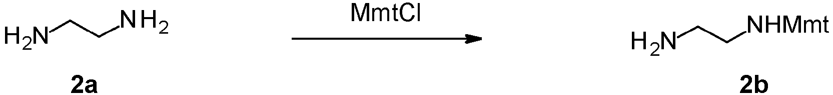

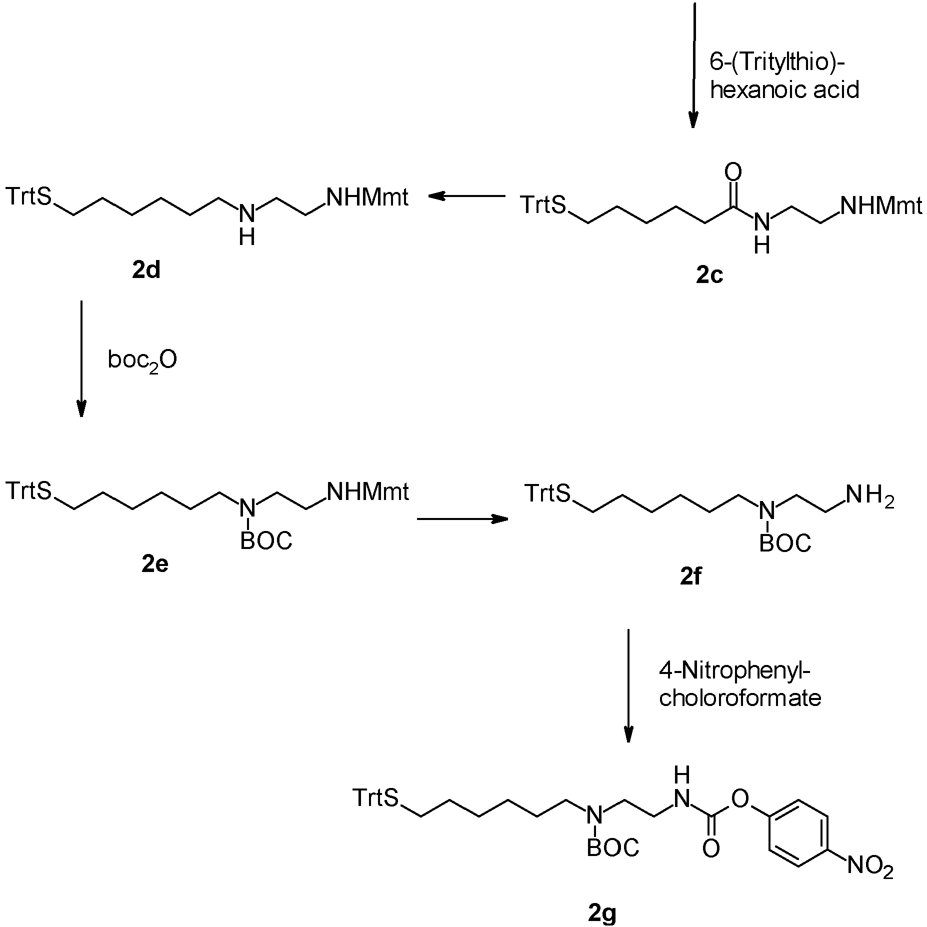

4-Methoxytriphenylmethyl chloride (3.00 g, 9.71 mmol) was dissolved in DCM (20 mL) and added dropwise under stirring to a solution of ethylenediamine **2a** (6.5 mL, 97.3 mmol) in DCM (20 mL). The reaction mixture was stirred for 2 h at rt after which it was diluted with diethyl ether (300 mL), washed 3 x with brine/0.1 M NaOH 30/1 (v/v) and once with brine. The organic phase was dried over Na₂SO₄ and **2b** was isolated upon evaporation of the solvent.

| | |
|---|---|
| Yield: | 3.18 g (98%) |

Mmt protected intermediate **2b** (3.18 g, 9.56 mmol) was dissolved in DCM (30 mL). 6-(Tritylthio)-hexanoic acid (4.48 g, 11.5 mmol), PyBOP (5.67 g, 10.9 mmol) and DIPEA (5.0 mL, 28.6 mmol) were added and the mixture was stirred for 30 min at rt. The solution was diuted with diethyl ether (250 mL), washed 3 x with brine/0.1 M NaOH 30/1 (v/v) and once with brine. The organic phase was dried over Na₂SO₄ and the solvent was removed in vacuo. **2c** was purified using flash chromatography.

| | |
|---|---|
| Yield: | 5.69 g (85 %) |
| MS: | m/z 705.4 = [M+H]⁺, (calculated monoisotopic mass = 704.34). |

Compound **2c** (3.19 g, 4.53 mmol) was dissolved in abhydrous THF (50 mL), 1 M BH₃·THF solution in THF (8.5 mL, 8.5 mmol) was added and the mixture was stirred for 16 h at rt. More 1 M BH₃·THF solution in THF (14 mL, 14.0 mmol) was added and the mixture was stirred for further 16 h at rt. Methanol (8.5 mL) and N,N'-dimethyl-ethylendiamine (3.00 mL, 27.9 mmol) were added and the mixture was heated under reflux for 3 h. The mixture was allowed to cool down and ethyl acetate (300 mL) was added. The solution was washed 2 x with aqueous Na₂CO₃ and 2 x with aqueous NaHCO₃. The organic phase was dried over Na₂SO₄ and the solvent was removed in vacuo to obtain **2d.**

| | |
|---|---|
| Yield: | 3.22 g (103 %) |
| MS: | m/z 691.4 = [M+H]⁺, (calculated monoisotopic mass = 690.36). |

Di-tert-butyl dicarbonate (2.32 g, 10.6 mmol) and DIPEA (3.09 mL, 17.7 mmol) were dissolved in DCM (5 mL) and added to a solution of **2d** (2.45 g, 3.55 mmol) in DCM (5 mL). The mixture was stirred for 30 min at rt. The solution was concentrated in vacuo and purified by flash chromatography to obtain product **2e.**

| | |
|---|---|
| Yield: | 2.09 g (74 %) |
| MS: | m/z 791.4 = [M+H]⁺, (calculated monoisotopic mass = 790.42). |

Compound **2e** (5.01 g, 6.34 mmol) was dissolved in acetonitrile (80 mL). 0.4 M aqueous HCl (80 mL) followed by acetonitrile (20 mL) was added and the mixture was stirred for 1 h at rt. The pH was adjusted to pH 5.5 by addition of aqueous 5 M NaOH. The organic solvent was removed in vacuo and the remaining aqueous solution was extracted 4 x with DCM. The combined organic phases were dried over Na₂SO₄ and the solvent was removed in vacuo to obtain product **2f.**

| | |
|---|---|
| Yield: | 4.77 g (95 %) |
| MS: | m/z 519.3 = [M+H]⁺, (calculated monoisotopic mass = 518.30). |

Compound **2f** (5.27 g, 6.65 mmol) was dissolved in DCM (30 mL) and added to a solution of p-nitrophenyl chloroformate (2.01 g, 9.98 mmol) in DCM (25 mL). 2,4,6-trimethylpyridine (4.38 mL, 33.3 mmol) was added and the solution was stirred for 45 min at rt. The solution was concentrated *in vacu* and purified by flash chromatography to obtain product **2g.**

| | |
|---|---|
| Yield: | 4.04 g (89 %) |
| MS: | m/z 706.32 = [M+Na]⁺, (calculated monoisotopic mass = 683.30). |

### Example 3

### Synthesis of permanent S1 PTH(1-34) conjugate 3 (not according to the invention)

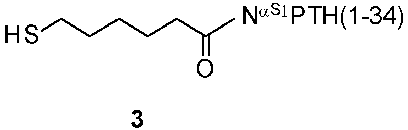

Side chain protected PTH(1-34) on TCP resin having Fmoc protected N-terminus was Fmoc deprotected according to the procedure given in Materials and Methods. A solution of 6-tritylmercaptohexanoic acid (62.5 mg, 160 µmol), PyBOP (80.1 mg, 154 µmol) and DIPEA (53 µL, 306 µmol) in DMF (2 mL) was added to 0.21 g (51 µmol) of the resin. The suspension was agitated for 80 min at rt. The resin was washed 10 x with DMF, 10 x with DCM and dried *in vacuo.* Cleavage of the peptide from the resin and removal of protecting groups was achieved by adding 10 mL cleavage cocktail 100/3/3/2/1 (v/w/v/v/v) TFA/DTT/TES/water/thioanisole and agitating the suspension for 1 h at rt. Crude 3 was precipitated in pre-cooled diethyl ether (-18 °C). The precipitate was dissolved in ACN/water and purified by RP-HPLC. The product fractions were freeze-dried.

| | |
|---|---|
| Yield: | 36 mg (14 %), **3***8 TFA |
| MS: | m/z 1062.31 = [M+4H]⁴⁺, (calculated monoisotopic mass for [M+4H]⁴⁺ = 1062.30). |

### Example 4

### Synthesis of permanent K26 PTH(1-34) conjugate 4 (not according to the invention)

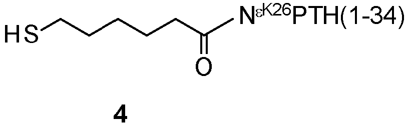

Side chain protected PTH(1-34) on TCP resin having Boc protected N-terminus and ivDde protected side chain of Lys26 was ivDde deprotected according to the procedure given in Materials and Methods. A solution of 6-tritylmercaptohexanoic acid (107 mg, 273 µmol), PyBOP (141 mg, 273 µmol) and DIPEA (95 µL, 545 µmol) in DMF (3 mL) was added to 0.80 g (90.9 µmol) of the resin. The suspension was agitated for 1 h at rt. The resin was washed 10 x with DMF, 10 x with DCM and dried *in vacuo.* Cleavage of the peptide from the resin and removal of protecting groups was achieved by adding 6 mL cleavage cocktail 100/3/3/2/1 (v/w/v/v/v) TFA/DTT/TES/water/thioanisole and agitating the suspension for 1 h at rt. Crude **4** was precipitated in pre-cooled diethyl ether (-18 °C). The precipitate was dissolved in ACN/water and purified by RP-HPLC. The product fractions were freeze-dried.

| | |
|---|---|
| Yield: | 40 mg (8 %), **4***8 TFA |
| MS: | m/z 1062.30 = [M+4H]⁴⁺, (calculated monoisotopic mass for [M+4H]⁴⁺ = 1062.30). |

### Example 5

### Synthesis of transient S1 PTH(1-34) conjugate

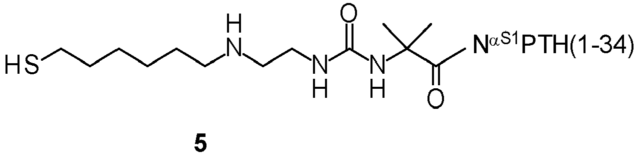

Side chain protected PTH(1-34) on TCP resin having Fmoc protected N-terminus was Fmoc deprotected according to the procedure given in Materials and Methods. A solution of Fmoc-Aib-OH (79 mg, 244 µmol), PyBOP (127 mg, 244 µmol) and DIPEA (64 µL, 365 µmol) in DMF (1.5 mL) was added to 0.60 g (61 µmol) of the resin. The suspension was agitated for 16 h at rt. The resin was washed 10 x with DMF and Fmoc-deprotected as described above. A solution of **2g** (167 mg, 244 µmol) and DIPEA (64 µL, 365 µmol) in DMF (1.5 mL) was added to the resin. The suspension was agitated for 24 h at rt. The resin was washed 10 x with DMF, 10 x with DCM and dried *in vacuo.* Cleavage of the peptide from the resin and removal of protecting groups was achieved by adding 7 mL cleavage cocktail 100/3/3/2/1 (v/w/v/v/v) TFA/DTT/TES/water/thioanisole and agitating the suspension for 1 h at rt. Crude 5 was precipitated in pre-cooled diethyl ether (-18 °C). The precipitate was dissolved in ACN/water and purified by RP-HPLC. The product fractions were freeze-dried.

| | |
|---|---|
| Yield: | 78 mg (24 %), **5***9 TFA |
| MS: | m/z 1101.59 = [M+4H]⁴⁺, (calculated monoisotopic mass for [M+4H]⁴⁺ = 1101.57). |

### Example 6

### Synthesis of transient S1 PTH(1-34) conjugate 6 (not according to the invention)

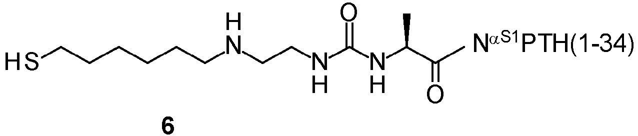

Side chain protected PTH(1-34) on TCP resin having Fmoc protected N-terminus was Fmoc deprotected according to the procedure given in Materials and Methods. A solution of Fmoc-Ala-OH (32 mg, 102 µmol), PyBOP (53 mg, 102 µmol) and DIPEA (27 µL, 152 µmol) in DMF (3 mL) was added to 0.25 g (25 µmol) of the resin. The suspension was shaken for 1 h at rt. The resin was washed 10 x with DMF, 10 x with DCM and dried under vacuum. Fmoc-deprotection was performed as described above. A solution of **2g** (69 mg, 102 µmol) and DIPEA (27 µL, 152 µmol) in DMF (3 mL) was added to the resin. The suspension was agitated for 1.5 h at rt. The resin was washed 10 x with DMF, 10 x with DCM and dried *in vacuo.* Cleavage of the peptide from the resin and removal of protecting groups was achieved by adding 3 mL cleavage cocktail 100/3/3/2/1 (v/w/v/v/v) TFA/DTT/TES/water/thioanisole and agitating the suspension for 1 h at rt. Crude **6** was precipitated in pre-cooled diethyl ether (-18 °C). The precipitate was dissolved in ACN/water and purified by RP-HPLC. The product fractions were freeze-dried.

| | |
|---|---|
| Yield: | 25 mg (18 %), **6***9 TFA |
| MS: | m/z 1098.75 = [M+4H]⁴⁺, (calculated monoisotopic mass for [M+4H]⁴⁺ = 1098.07). |

### Example 7

### Synthesis of transient S1 PTH(1-34) conjugate 7 (not according to the invention)

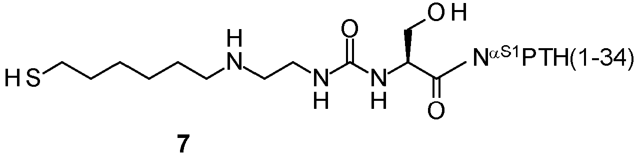

Side chain protected PTH(1-34) on TCP resin having Fmoc protected N-terminus was Fmoc deprotected according to the procedure given in Materials and Methods. A solution of Fmoc-Ser(Trt)-OH (117 mg, 205 µmol), PyBOP (108 mg, 207 µmol) and DIPEA (53 µL, 305 µmol) in DMF (2 mL) was added to 0.50 g (51 µmol) of the resin. The suspension was agitated for 1 h at rt. The resin was washed 10 x with DMF, 10 x with DCM and dried under vacuum. Fmoc-deprotection was performed as described above. A solution of **2g** (144 mg, 211 µmol) and DIPEA (53 µL, 305 µmol) in DMF (1.8 mL) was added to the resin. The suspension was shaken for 7 h at rt. The resin was washed 10 x with DMF, 10 x with DCM and dried *in vacuo.* Cleavage of the peptide from the resin and removal of protecting groups was achieved by adding 6 mL cleavage cocktail 100/3/3/2/1 (v/w/v/v/v) TFA/DTT/TES/water/thioanisole and agitating the suspension for 1 h at rt. Crude 7 was precipitated in pre-cooled diethyl ether (-18 °C). The precipitate was dissolved in ACN/water and purified by RP-HPLC. The product fractions were freeze-dried.

| | |
|---|---|
| Yield: | 54 mg (20 %), **7***9 TFA |
| MS: | m/z 1102.08 = [M+4H]⁴⁺, (calculated monoisotopic mass for [M+4H]⁴⁺ = 1102.07). |

### Example 8

### Synthesis of transient S1 PTH(1-34) conjugate 8 (not according to the invention)

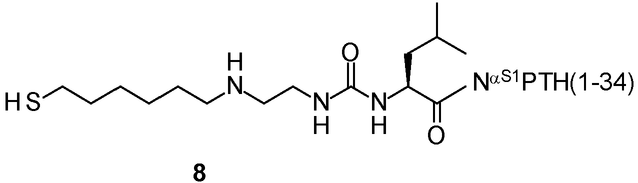

Side chain protected PTH(1-34) on TCP resin having Fmoc protected N-terminus was Fmoc deprotected according to the procedure given in Materials and Methods. A solution of Fmoc-Leu-OH (36 mg, 102 µmol), PyBOP (53 mg, 102 µmol) and DIPEA (27 µL, 152 µmol) in DMF (3 mL) was added to 0.25 g (25 µmol) of the resin. The suspension was agitated for 1 h at rt. The resin was washed 10 x with DMF, 10 x with DCM and dried under vacuum. Fmoc-deprotection was performed as described above. A solution of **2g** (69 mg, 102 µmol) and DIPEA (27 µL, 152 µmol) in DMF (3 mL) was added to the resin. The suspension was agitated for 1.5 h at rt. The resin was washed 10 x with DMF, 10 x with DCM and dried *in vacuo.* Cleavage of the peptide from the resin and removal of protecting groups was achieved by adding 3 mL cleavage cocktail 100/3/3/2/1 (v/w/v/v/v) TFA/DTT/TES/water/thioanisole and agitating the suspension for 1 h at rt. Crude **8** was precipitated in pre-cooled diethyl ether (-18 °C). The precipitate was dissolved in ACN/water and purified by RP-HPLC. The product fractions were freeze-dried.

| | |
|---|---|
| Yield: | 31 mg (22 %), **8***9 TFA |
| MS: | m/z 1109.32 = [M+4H]⁴⁺, (calculated monoisotopic mass for [M+4H]⁴⁺ = 1108.58). |

### Example 9

### Synthesis of transient S1 PTH(1-34) conjugate 9 (not according to the invention)

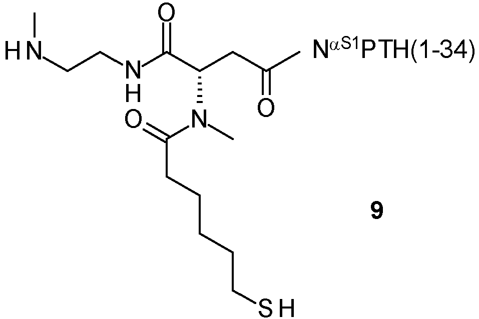

Side chain protected PTH(1-34) on TCP resin having Fmoc protected N-terminus was Fmoc deprotected according to the procedure given in Materials and Methods. A solution of **1e** (182 mg, 213 µmol), PyBOP (111 mg, 213 µmol) and DIPEA (93 µL, 532 µmol) in DMF (5 mL) was added to 2.00 g (107 µmol) of the resin. The suspension was agitated for 16 h at rt. The resin was washed 10 x with DMF, 10 x with DCM and dried under vacuum. Cleavage of the peptide from the resin and removal of protecting groups was achieved by adding 20 mL cleavage cocktail 100/3/3/2/1 (v/w/v/v/v) TFA/DTT/TES/water/thioanisole and agitating the suspension for 1 h at rt. Crude **9** was precipitated in pre-cooled diethyl ether (-18 °C). The precipitate was dissolved in ACN/water and purified by RP-HPLC. The product fractions were freeze-dried.

| | |
|---|---|
| Yield: | 47 mg (8 %), **9***9 TFA |
| MS: | m/z 1108.58 = [M+4H]⁴⁺, (calculated monoisotopic mass for [M+4H]⁴⁺ = 1108.57). |

### Example 10

### Synthesis of transient K26 PTH(1-34) conjugate 10 (not according to the invention)

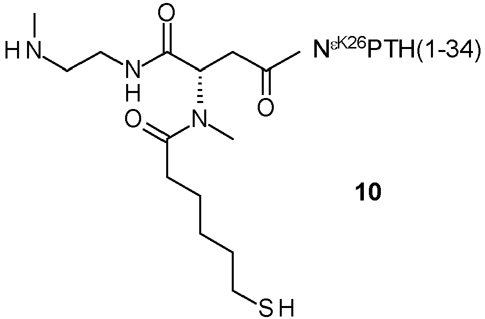

Side chain protected PTH(1-34) on TCP resin having Boc protected N-terminus and ivDde protected side chain of Lys26 was ivDde deprotected according to the procedure given in Materials and Methods. A solution of **1f** (867 mg, 910 µmol) and DIPEA (0.24 mL, 1.36 mmol) in DMF (5 mL) was added to 1.91 g (227 µmol) of the resin. The suspension was agitated for 1 h at rt. The resin was washed 10 x with DMF, 10 x with DCM and dried under vacuum. Cleavage of the peptide from the resin and removal of protecting groups was achieved by adding 20 mL cleavage cocktail 100/3/3/2/1 (v/w/v/v/v) TFA/DTT/TES/water/thioanisole and shaking the suspension for 1 h at rt. Crude **10** was precipitated in pre-cooled diethyl ether (-18 °C). The precipitate was dissolved in ACN/water and purified by RP-HPLC. The product fractions were freeze-dried.

| | |
|---|---|
| Yield: | 92 mg (7 %), **10***9 TFA |
| MS: | m/z 1108.58 = [M+4H]⁴⁺, (calculated monoisotopic mass for [M+4H]⁴⁺ = 1108.57). |

### Example 11

### Synthesis of low molecular weight transient S1 PEG conjugate 11b (not according to the invention)

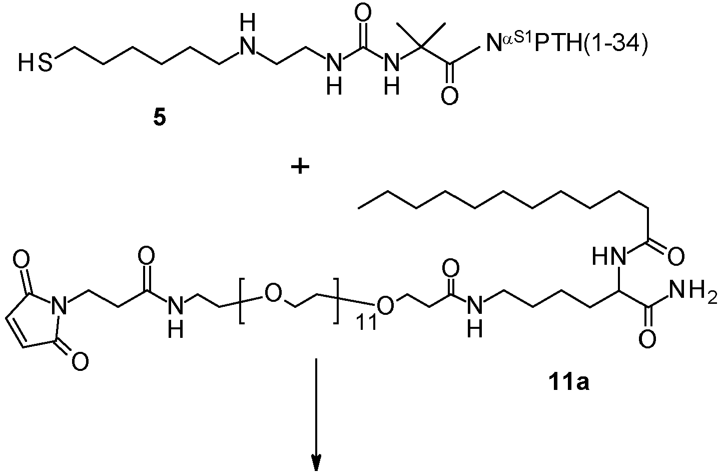

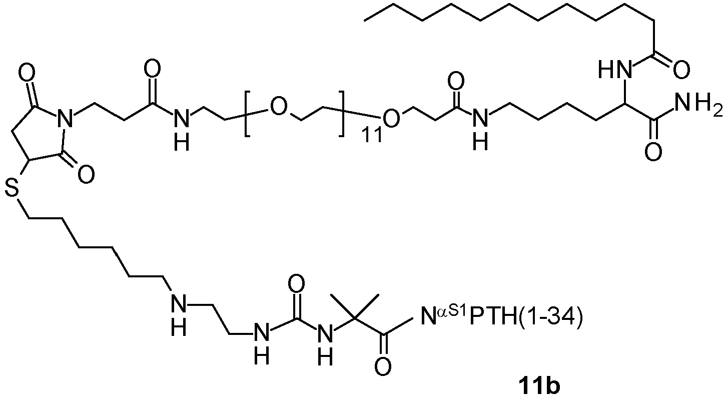

0.15 mL of a 0.5 M NaH₂PO₄ buffer (pH 7.4) was added to 0.5 mL of a 20 mg/mL solution of thiol **5** (10 mg, 1.84 µmol) in 1/1 (v/v) acetonitrile/water containing 0.1 % TFA (v/v). The solution was incubated at rt for 10 min after which 238 µL of a 10 mg/mL solution of maleimide **11a** (2.4 mg, 2.21 µmol) in 1/1 (v/v) acetonitrile/water containing 0.1 % TFA (v/v) were added. The solution was incubated for 20 min at rt. 10 µL TFA was added and the mixture was purified by RP-HPLC. The product fractions were freeze-dried to obtain **11b.**

| | |
|---|---|
| Yield: | 3.1 mg (26 %), **11b***9 TFA |
| MS: | m/z 1097.00 = [M+4H]⁴⁺, (calculated monoisotopic mass for [M+5H]⁵⁺ = 1096.99). |

### Example 12

### Synthesis of low molecular weight transient S1 PEG conjugate 12 (not according to the invention)

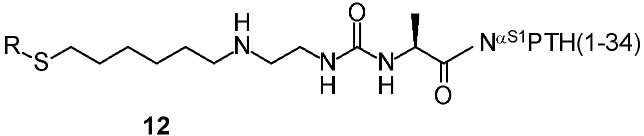

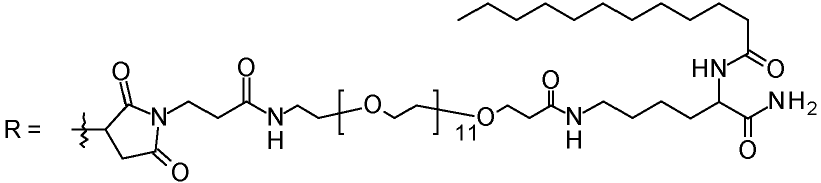

Conjugate **12** was synthesized as described for **11b** by using thiol **6** (10 mg, 1.85 µmol) and maleimide **11a** (2.4 mg, 2.21 µmol).

| | |
|---|---|
| Yield: | 10 mg (83 %), **12***9 TFA |
| MS: | m/z 1094.20 = [M+4H]⁴⁺, (calculated monoisotopic mass for [M+4H]⁴⁺ = 1094.19). |

### Example 13

### Synthesis of low molecular weight transient S1 PEG conjugate 13 (not according to the invention)

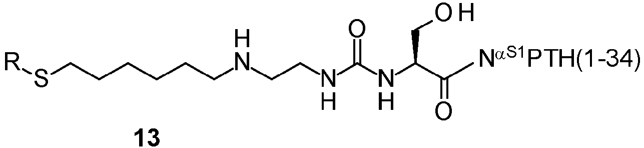

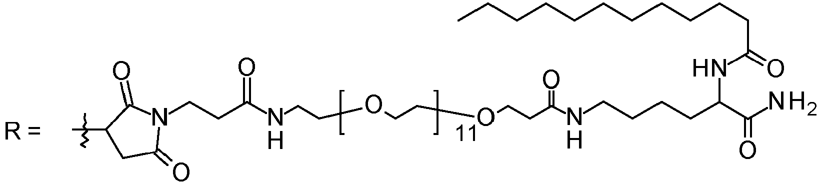

Conjugate **13** was synthesized as described for **11b** by using thiol **7** (10 mg, 1.84 µmol) and maleimide **11a** (2.4 mg, 2.21 µmol).

| | |
|---|---|
| Yield: | 8 mg (67 %), **13***9 TFA |
| MS: | m/z 1097.40 = [M+5H]⁵⁺, (calculated monoisotopic mass for [M+5H]⁵⁺ = 1097.39). |

### Example 14

### Synthesis of low molecular weight transient S1 PEG conjugate 14 (not according to the invention)

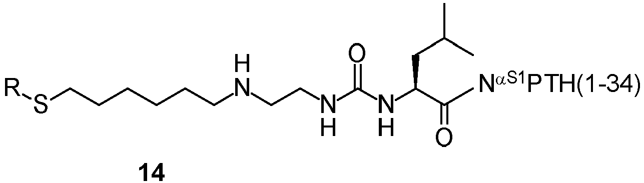

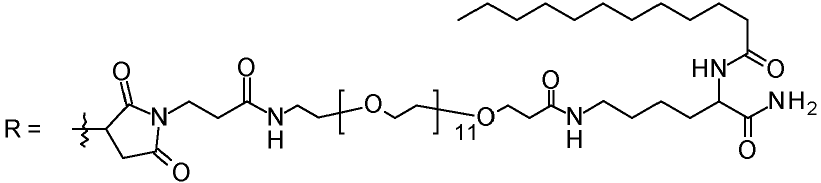

Conjugate **14** was synthesized as described for **11b** by using thiol **8** (10 mg, 1.83 µmol) and maleimide **11a** (2.4 mg, 2.21 µmol).

| | |
|---|---|
| Yield: | 4 mg (33 %), **14***9 TFA |
| MS: | m/z 1378.01 = [M+4H]⁴⁺, (calculated monoisotopic mass for [M+4H]⁴⁺ = 1378.00). |

### Example 15

### Synthesis of low molecular weight transient K26 PEG conjugate 15 (not according to the invention)

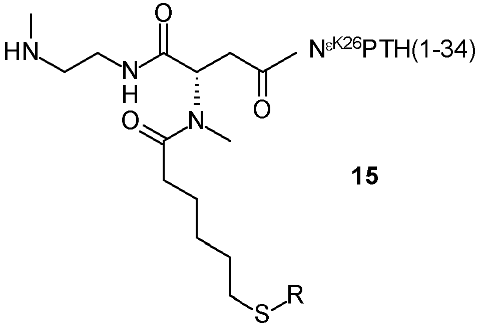

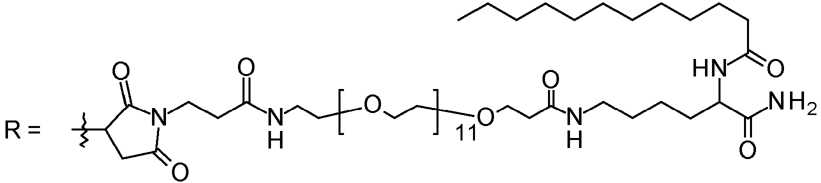

Conjugate **15** was synthesized as described for **11b** by using thiol **10** (5.2 mg, 0.95 µmol) and maleimide **11a** (1.23 mg, 1.14 µmol).

| | |
|---|---|
| Yield: | 2.1 mg (33 %), **15***9 TFA |
| MS: | m/z 1102.60 = [M+5H]⁵⁺, (calculated monoisotopic mass for [M+5H]⁵⁺ = 1102.59). |

### Example 16

### Synthesis of permanent 2x20 kDa S1 PEG conjugate 16 (not according to the invention)

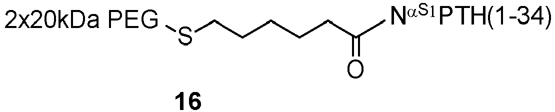

772 µL of a solution containing thiol **3** (19.4 mg/mL, 15 mg, 3.54 µmol) and 2.5 mg/mL Boc-L-Met in 1/1 (v/v) acetonitrile/water containing 0.1 % TFA (v/v) were added to 1.87 mL of a solution containing PEG 2x20 kDa maleimide (Sunbright GL2-400MA, 187 mg, 4.32 µmol) and 2.5 mg/mL Boc-L-Met in water containing 0.1 % TFA (v/v). 0.5 M NaH₂PO₄ buffer (0.66 mL, pH 7.0) was added and the mixture was stirred for 30 min at rt. 10 µL of a 270 mg/mL solution of 2-mercaptoethanol in water was added. The mixture was stirred for 5 min at rt and 0.33 mL 1 M HCl were added. Conjugate **16** was purified by IEX followed by RP-HPLC using a linear gradient of solvent system A (water containing 0.1 % AcOH v/v) and solvent system B (acetonitrile containing 0.1 % AcOH v/v). The product containing fractions were freeze-dried.

| | |
|---|---|
| Yield: | 97 mg (2.01 µmol, 57 %) conjugate **16***8 AcOH |

### Example 17

### Synthesis of permanent 2x20 kDa K26 PEG conjugate 17 (not according to the invention)

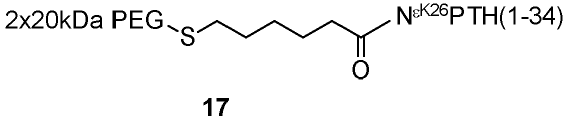

Conjugate **17** was prepared as described for **16** by reaction of thiol **4** (15 mg, 3.53 µmol) and PEG 2x20 kDa maleimide (Sunbright GL2-400MA, 187 mg, 4.32 µmol).

| | |
|---|---|
| Yield: | 80 mg (1.79 µmol, 51 %) conjugate **17***8 AcOH |

### Example 18

### Synthesis of transient 2x20 kDa S1 PEG conjugate 18

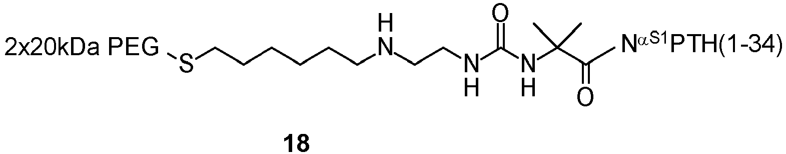

Conjugate **18** was prepared as described for **16** by reaction of thiol **5** (37 mg, 8.40 µmol) and PEG 2x20 kDa maleimide (Sunbright GL2-400MA, 445 mg, 9.24 µmol).The reaction was quenched by addition of 50 µL TFA without prior addition of 2-mercaptoethanol. Conjugate **18** was purified by IEX followed by SEC for desalting. The product containing fractions were freeze-dried.

| | |
|---|---|
| Yield: | 161 mg (3.33 µmol, 40 %) conjugate **18***9 AcOH |

### Example 19

### Synthesis of transient 2x20 kDa S1 PEG conjugate 19 (not according to the invention)

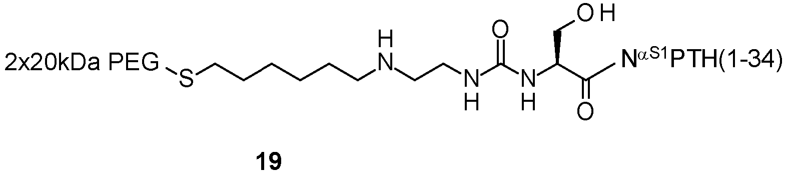

Conjugate **19** was prepared as described for **16** by reaction of thiol 7 (27 mg, 6.14 µmol) and PEG 2x20 kDa maleimide (Sunbright GL2-400MA, 325 mg, 7.50 µmol).

| | |
|---|---|
| Yield: | 249 mg (5.16 µmol, 84 %) conjugate **19***9 AcOH |

### Example 20

### Synthesis of transient 2x20 kDa S1 PEG conjugate 20 (not according to the invention)

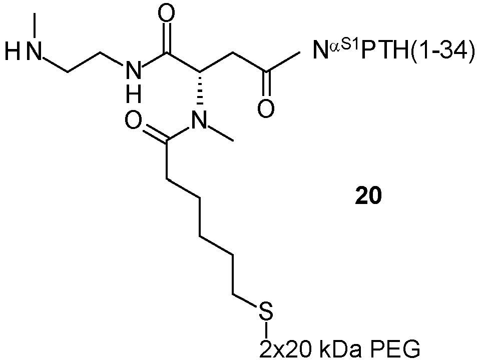

Conjugate **20** was prepared as described for **16** by reaction of thiol **9** (38 mg, 8.59 µmol) and PEG 2x20 kDa maleimide (Sunbright GL2-400MA, 455 mg, 9.45 µmol).The reaction was quenched by addition of 50 µL TFA without prior addition of 2-mercaptoethanol. Conjugate **20** was purified by IEX followed by SEC for desalting. The product containing fractions were freeze-dried.

| | |
|---|---|
| Yield: | 194 mg (4.01 µmol, 47 %) conjugate **20***9 AcOH |

### Example 21

### Synthesis of transient 2x20 kDa K26 PEG conjugate 21 (not according to the invention)

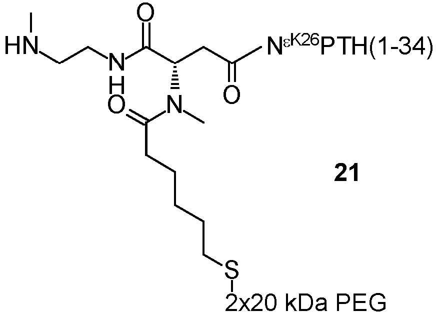

Conjugate **21** was prepared as described for **16** by reaction of thiol **10** (34 mg, 7.58 µmol) and PEG 2x20 kDa maleimide (Sunbright GL2-400MA, 401 mg, 9.26 µmol).

| | |
|---|---|
| Yield: | 256 mg (5.30 µmol, 70 %) conjugate **21***9 AcOH |

### Example 22

### In vitro release kinetics of transient low molecular weight PEG conjugates (not according to the invention)

Conjugates **11b, 12, 13, 14,** and **15** were dissolved in pH 7.4 phosphate buffer (60 mM NaH₂PO₄, 3 mM EDTA, 0.01 % Tween-20, adjusted to pH 7.4 by NaOH) containing 0.05 mg/mL pentafluorophenol as internal standard at a concentration of approximately 1 mg conjugate/mL. The solutions were filtered sterile and incubated at 37°C. At time points, aliquots were withdrawn and analysed by RP-HPLC and ESI-MS. The fraction of released PTH at a particular time point was calculated from the ratio of UV peak areas of liberated PTH and PEG conjugate. The % released PTH was plotted against incubation time. Curve-fitting software was applied to calculate the corresponding half times of release.

### Results:

For conjugate **11b** a release half life time of 3.2 d was obtained.

For conjugate **12** a release half life time of 8.7 d was obtained.

For conjugate **13** a release half life time of 10.8 d was obtained.

For conjugate **14** a release half life time of 25.3 d was obtained.

For conjugate **15** a release half life time of 6.9 d was obtained.

### Example 23 (partially according to the invention)

### In vitro release kinetics of transient 2x20 kDa PEG conjugates

Conjugates **18, 19, 20,** and **21** were dissolved in pH 7.4 phosphate buffer (60 mM NaH₂PO₄, 3 mM EDTA, 0.01 % Tween-20, adjusted to pH 7.4 by NaOH) containing 0.08 mg/mL pentafluorophenol as internal standard at a concentration of approximately 5 mg conjugate/mL. The solutions were filtered sterile and incubated at 37°C. At time points, aliquots were withdrawn and analysed by RP-HPLC. The fraction of released PTH at a particular time point was calculated from the ratio of UV peak areas of liberated PTH and PEG conjugate. The % released PTH was plotted against incubation time. Curve-fitting software was applied to calculate the corresponding half times of release.

### Results:

For conjugate **18** a release half life time of 2.8 d was obtained.

For conjugate **19** a release half life time of 13.4 d was obtained.

For conjugate **20** a release half life time of 1.3 d was obtained

For conjugate **21** a release half life time of 7.1 d was obtained

### Example 24 (not according to the invention)

### PTH receptor activity of permanent 2x20 kDa PEG conjugates 16 and 17 in cell based assay

The residual PTH activity of permanently PEGylated conjugates **16** and **17** was quantified by measuring cAMP production from HEK293 cells over-expressing the PTH/PTHrP1 receptor (Hohenstein A, Hebell M, Zikry H, El Ghazaly M, Mueller F, Rohde, J. Development and validation of a novel cell-based assay for potency determination of human parathyroid hormone (PTH), Journal of Pharmaceutical and Biomedical Analysis September 2014, 98: 345-350). PTH(1-34) from NIBSC (National Institute for Biological Standards and Control, UK) was used as reference standard.

### Results:

For conjugate **16** a receptor activity of 0.12 % was found relative to PTH(1-34) reference

For conjugate **17** a receptor activity of 0.11 % was found relative to PTH(1-34) reference

The results indicate an effective lowering of receptor activity in the permanent 2x20 kDa PEG conjugates **16** and **17.** It can be concluded that similar conjugates with transiently Ser1 or Lys26 linked PTH (like e.g. **18** and **21**) are suitable PTH prodrugs providing low residual receptor activity. Direct analysis of transient conjugates in the cell assay is not possible due to linker cleavage under the assay conditions. The released PTH would influence the assay result.

### Example 25 (not according to the invention)

### Pharmacokinetic study of permanent 2x20 kDa PEG conjugates 16 and 17 in rats

Male Wistar rats (6 weeks, 230-260 g) received either a single intravenous (2 groups, n = 3 animals each) or a single subcutaneous (2 groups, n = 3 animals each) administration of **16** or **17** at doses of 29 µg/rat PTH_{eq} and 31 µg/rat PTH_{eq} respectively. Blood samples were collected up to 168 h post dose, and plasma was generated. Plasma PTH(1-34) concentrations were determined by quantification of the N-terminal signature peptide (sequence: IQLMHNLGK) and the C-terminal signature peptide (sequence: LQDVHNF) after LysC and GluC digestion as described in Materials and Methods.

Results: Dose administrations were well tolerated with no visible signs of discomfort during administration and following administration. No dose site reactions were observed any time throughout the study. After intraveneous injection of **16** and **17** the total PTH(1-34) tₘₐₓ was observed at 15 min (earliest time point analyzed), followed by a slow decay in total PTH(1-34) content with a half life time of approx. 13 h and 11 h respectively. After subcutaneous injection the total PTH(1-34) concentration peaked at a tₘₐₓ of 24 h for both **16** and **17,** followed by a slow decay in total PTH(1-34) content with half life times of approx. 1.5 days for both conjugates. The bioavailability was approx. 40 % and 60 % respectively. Similar PK curves were obtained for the N- and the C-terminal signature peptide up to 168 h post dose, indicating the presence of intact PTH(1-34) in the conjugate.

The favourable long lasting PK and the stability of PTH in the conjugates indicate the suitability of the permanent 2x20 kDa PEG model compounds as slow releasing PTH prodrugs after subcutaneous injection. It can be concluded that similar conjugates with transiently Ser1 (like e.g. **18**) or Lys26 linked PTH are suitable PTH prodrugs providing long lasting levels of released bioactive PTH.

### Example 26 (not according to the invention)

### Pharmacokinetic study of transient 2x20 kDa S1 PEG conjugate 19 in cynomolgus monkeys

Male non naive cynomolgus monkeys (2-4years, 3.7-5.4 kg) received a single subcutaneous (n = 3 animals) administration of **19** at a dose of 70 µg/kg PTH_{eq}. Blood samples were collected up to 504 h post dose, and plasma was generated. Total plasma PTH(1-34) concentrations were determined by quantification of the N-terminal signature peptide (sequence: IQLMHNLGK) and the C-terminal signature peptide (sequence: LQDVHNF) after LysC and GluC digestion as described in Materials and Methods. The PEG concentrations were determined using the method described in Materials and Methods.

Results: Dose administrations were well tolerated with no visible signs of discomfort during administration. One animal showed showed visible signs of discomfort 72 h post dose, but recovered the days after. No dose site reactions were observed any time throughout the study. The total PTH(1-34) concentration peaked at a tₘₐₓ of 24 h, followed by a slow decay in total PTH(1-34) content with a half life time of approx. 2.5 d for the N-terminal signature peptide and 0.9 d for the C-terminal signature peptide. The PEG concentration peaked at tₘₐₓ of 24 h, followed by a slow decay in PEG concentration with a half life time of 3.5 d.

It can be concluded that conjugate **19** is a suitable prodrug for sustained delivery of PTH.

### Example 27

### Pharmacokinetic study of transient 2x20 kDa S1 PEG conjugate 18 in cynomolgus monkeys

Non naive cynomolgus monkeys (2-3 years, 2.5-4 kg) received daily subcutaneous (n = 2 animals - 1 male / 1 female) administration of **18** at dose levels of 0.2, 0.5, and 1 µg/kg PTH_{eq} for 28 days. Blood samples were collected up to 28 days (at days 1, 13, and, 27 samples were collected at pre-dose, 2 h, 4 h, 8 h, 12 h, and 24 h post-dose) and plasma was generated. Plasma PTH(1-34) concentrations were determined by quantification of the N-terminal signature peptide (sequence: IQLMHNLGK) and the C-terminal signature peptide (sequence: LQDVHNF) after LysC and GluC digestion as described in Materials and Methods.

Results: All dose administrations were performed without incident. No dose site reactions were observed any time throughout the study. Dose linearity was observed in the three groups. Dose stacking was observed from day 1 compared with day 13 and day 27. Total PTH(1-34) concentrations were quantified via the N-terminal signature peptide (sequence: IQLMHNLGK) at steady state (during day 27).

A low peak-to-trough ratio of total PTH(1-34) for all dose groups of below 3 was observed after daily subcutaneous application at steady state in cynomolgus monkeys. As free peptide concentrations at steady state are correlated to total PTH(1-34) concentration, the peak-to-trough ratio for the free peptide is below 4 in cynomolgus monkeys.

### Example 28

### Pharmacokinetic study of transient 2x20 kDa S1 PEG conjugate 18 in cynomolgus monkeys

Naive cynomolgus monkeys (2-3.5 years, 2-5 kg) (3-5 males / 3-5 females) received daily subcutaneous administrations of **18** at dose levels of 0.2, 0.5 and 1.5 µg PTH/kg. Blood samples were collected at; Day 1: pre-dose, 4 h, 8 h, 12 h, 18 h, and 24 h post-dose, at Day 8: pre-dose, at Day 14: predose, 8h, and 12 h and at Day 28: 3 h, 6 h, 8 h, 12 h, 18 h, 24 h, 72 h, 168 h, and 336 h) and plasma was generated. Total PTH plasma concentrations were determined by quantification of the N-terminal signature peptide (sequence: IQLMHNLGK) after LysC and GluC digestion as presented earlier in Materials and Methods.

Results: Systemic exposure expressed as Cₘₐₓ and AUC increased in an approximately dose proportional manner. Systemic exposure of Total PTH expressed as AUC accumulated approximately 3-fold from Day 1 to Day 28.

A low mean peak-to-trough ratio of Total PTH for all dose groups of 1.5 was observed after daily subcutaneous administration in cynomolgus monkeys at Day 28 (steady state observed from Day 8). This low mean peak-to-trough ratio of Total PTH can also be translated to Free PTH.

### Example 29

### Pharmacokinetic study of transient 2x20 kDa S1 PEG conjugate 18 in Sprague-Dawley rats

Sprague-Dawley Crl:CD(SD) rats (initiation of dosing at 8 weeks of age) received daily subcutaneous administrations of **18** at dose levels of 10, 30 and 60 µg PTH/kg for 28 days. A TK group containing of 9 males and 9 females per dose group was divided into 3 subgroup with 3 rats per subgroup. Blood samples were collected up to 28 days with 3 rats per sex, per sampling time point. Samples were collected at Day 1: pre-dose, 4 h, 8 h, 12 h, 18 h, and 24 h post-dose, and at Day 28: 3 h, 6 h, 8 h, 12 h, 18 h, 24 h, and 336 h and plasma was generated. The total PTH plasma concentrations were determined by quantification of the N-terminal signature peptide (sequence: IQLMHNLGK) after LysC and GluC digestion as presented earlier in Materials and Methods.

The free PTH plasma concentrations were determined by quantification as the sum of PTH(1-34) and PTH(1-33) by LC-MS/MS as presented earlier in Materials and Methods.

Results: Systemic exposure of total PTH and free PTH expressed as mean Cₘₐₓ and AUC increased in an approximately dose proportional manner. Systemic exposure of total PTH expressed as mean AUC accumulated 3-6 fold from day 1 to day 28 and free PTH expressed as mean AUC accumulated 2-3 fold from day 1 to day 28. Systemic exposure of total PTH in the female rat was approximately 2-fold higher than in males. Systemic exposure of free PTH was slightly higher in the female rat than in males.

A low mean peak-to-trough ratio of total PTH for all dose groups of 1.2 was observed after daily subcutaneous administration in Sprague-Dawley rats at day 28 (steady state observed from Day 8). A low mean peak-to-trough ratio of free PTH in the range 1.5 - 2.4 was observed after daily subcutaneous administration in Sprague-Dawley rats at day 28.

### Abbreviations:

- ACN: acetonitrile
- AcOH: acetic acid
- Aib: 2-aminoisobutyric acid
- BMD: bone mineral density
- Bn: benzyl
- Boc: tert-butyloxycarbonyl
- COMU: (1-cyano-2-ethoxy-2-oxoethylidenaminooxy)dimethylamino-morpholino-carbenium hexafluorophosphate
- cAMP: cyclic adenosine monophosphate
- d: day
- DBU: 1,3-diazabicyclo[5.4.0]undecene
- DCC: N,N'-dicyclohexylcarbodiimide
- DCM: dichloromethane
- DIPEA: N,N-diisopropylethylamine
- DMAP: dimethylamino-pyridine
- DMF: N,N-dimethylformamide
- DMSO: dimethylsulfoxide
- DTT: dithiothreitol
- EDTA: ethylenediaminetetraacetic acid
- eq: stoichiometric equivalent
- ESI-MS: electrospray ionization mass spectrometry
- Et: ethyl
- Fmoc: 9-fluorenylmethyloxycarbonyl
- Glu-C: endoproteinase Glu-C
- h: hour
- HATU: O-(7-azabenzotriazole-1-yl)-N,N,N',N'-tetramethyluronium hexafluorophosphate
- HPLC: high performance liquid chromatography
- ivDde: 4,4-dimethyl-2,6-dioxocyclohex-1-ylidene)-3-methylbutyl
- LC: liquid chromatography
- LTQ: linear trap quadrupole
- Lys-C: endoproteinase Lys-C
- LLOQ: lower limit of quantification
- Mal: 3-maleimido propyl
- Me: methyl
- MeOH: methanol
- min: minutes
- Mmt: monomethoxytrityl
- MS: mass spectrum / mass spectrometry
- m/z: mass-to-charge ratio
- OtBu: tert-butyloxy
- PEG: poly(ethylene glycol)
- pH: *potentia Hydrogenii*
- PK: pharmacokinetics
- Pr: propyl
- PTH: parathyroid hormone
- PyBOP: benzotriazol-1-yl-oxytripyrrolidinophosphonium hexafluorophosphate
- Q-TOF: quadrupole time-of-flight
- RP-HPLC: reversed-phase high performance liquid chromatography
- rt: room temperature
- SIM: single ion monitoring
- SEC: size exclusion chromatography
- sc: subcutaneous
- t_{1/2}: half life
- TCP: tritylchloride polystyrol
- TES: triethylsilane
- TFA: trifluoroacetic acid
- THF: tetrahydrofuran
- Tmob: 2,4,6-trimethoxybenzyl
- Trt: triphenylmethyl, trityl
- ULOQ: upper limit of quantification
- UPLC: ultra performance liquid chromatography
- UV: ultraviolet
- ZQ: single quadrupole

## Claims

1. A pharmaceutical composition comprising a PTH compound, wherein after subcutaneous administration to a non-human primate the pharmacokinetic profile of the PTH compound exhibits a peak to trough ratio of less than 4 within one injection interval, which is 24 hours, at steady state and wherein the PTH compound is a water-soluble compound of formula (Ia) or a pharmaceutically acceptable salt thereof 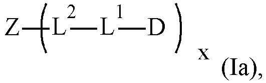 wherein
- D is a PTH moiety;
- L¹- is of formula (IIb-iii') 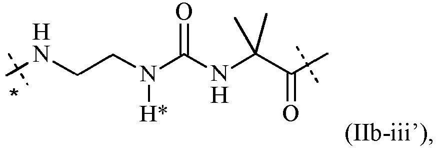 wherein
the dashed line indicates the attachment to the N-terminal amine functional group of -D through an amide bond;
the dashed line marked with the asterisk indicates attachment of -L²-Z; and
wherein -L¹- is optionally substituted, provided that the hydrogen marked with the asterisk in formula (IIb-iii') is not replaced by a substituent;
- L²- is a single chemical bond or a spacer moiety;
- Z is a water-soluble branched PEG-based carrier moiety with a molecular weight ranging from and including 15 kDa to 80 kDa;
x is 1;
and wherein the PTH compound has a residual PTH activity compared to the PTH activity of the corresponding free PTH of less than 1%.

2. The pharmaceutical composition of claim 1, wherein the subcutaneous administration occurs with a pen device.

3. The pharmaceutical composition of claim 1 or 2, wherein the peak to trough ratio is less than 3.

4. The pharmaceutical composition of any one of claims 1 to 3, wherein the residual PTH activity is less than 0.1%.

5. The pharmaceutical composition of any one of claims 1 to 4, wherein the pharmaceutical composition has a pH ranging from and including pH 3 to pH 8.

6. The pharmaceutical composition of any one of claims 1 to 5, wherein the pharmaceutical composition has a pH ranging from and including pH 4 to pH 5.

7. The pharmaceutical composition of any one of claims 1 to 6, wherein -D has the sequence of SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:107, SEQ ID NO:108, SEQ ID NO:109, SEQ ID NO:110, SEQ ID NO:111, SEQ ID NO:112, SEQ ID NO:113, SEQ ID NO:114 or SEQ ID NO:115.

8. The pharmaceutical composition of any one of claims 1 to 7, wherein -D has the sequence of SEQ ID NO:51.

9. The pharmaceutical composition of any one of claims 1 to 8, wherein -L²- is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl and C₂₋₅₀ alkynyl; wherein -T-, C₁₋₅₀ alkyl, C₂₋₅₀ alkenyl and C₂₋₅₀ alkynyl are optionally substituted with one or more -R^{y2}, which are the same or different, and wherein C₁₋₅₀ alkyl, C₂₋₅₀ alkenyl and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})- and -OC(O)N(R^{y3})-;
-R^{y1} and -R^{y1a} are independently of each other selected from the group consisting of -H, -T, C₁₋₁₀ alkyl, C₂₋₁₀ alkenyl and C₂₋₁₀ alkynyl; wherein -T, C₁₋₁₀ alkyl, C₂₋₁₀ alkenyl and C₂₋₁₀ alkynyl are optionally substituted with one or more -R^{y2}, which are the same or different, and wherein C₁₋₁₀ alkyl, C₂₋₁₀ alkenyl and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O) OR^{y5a}, -N(R^{y5})C(O)N(R^{y5a}R^{y5b}), -OC(O)N(R^{y5}R^{y5a}) and C₁₋₆ alkyl; wherein C₁₋₆ alkyl is optionally substituted with one or more halogen, which are the same or different; and each -R^{y3}, -R^{y3a}, -R^{y4}, -R^{y4a}, -R^{y5}, -R^{y5a} and -R^{y5b} is independently of each other selected from the group consisting of -H and C₁₋₆ alkyl; wherein C₁₋₆ alkyl is optionally substituted with one or more halogen, which are the same or different.

10. The pharmaceutical composition of any one of claims 1 to 9, wherein -L²- has a molecular weight ranging from 14 g/mol to 750 g/mol.

11. The pharmaceutical composition of any one of claims 1 to 10, wherein -Z has a molecular weight of about 40 kDa.

## Patentansprüche

1. Eine pharmazeutische Zusammensetzung enthaltend eine PTH-Verbindung, wobei nach subkutaner Verabreichung an einen nicht-humanen Primaten das pharmakokinetische Profil der PTH-Verbindung ein Hoch-zu-Tiefpunktverhältnis ("Peak to Trough Ratio") von weniger als 4 innerhalb eines Injektionsintervalls, welches 24 Stunden beträgt, im Steady-State aufweist und wobei die PTH-Verbindung eine wasserlösliche Verbindung der Formel (Ia) oder ein pharmazeutisch unbedenkliches Salz davon ist 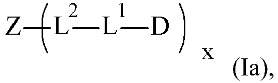 wobei
- D ein PTH-Rest ist;
- L¹- gemäß der Formel (IIb-iii') ist 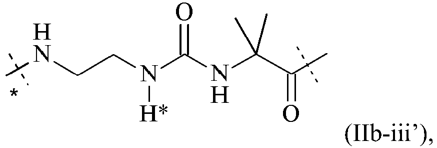 wobei
die gestrichelte Linie die Anknüpfung an die N-terminale funktionelle Amingruppe von -D mittels einer Amidbindung anzeigt;
die mit einem Asterisk markierte gestrichelte Linie Anknüpfung an -L²-Z anzeigt; und
wobei -L¹- optional substituiert ist, vorausgesetzt, dass der mit dem Asterisk markierte Wasserstoff in Formel (IIb-iii') nicht durch einen Substituenten ersetzt wird;
- L²- eine einzige chemische Bindung oder ein Spacer-Rest ist;
- Z ein wasserlöslicher verzweigter, PEG-basierter Trägerrest mit einem Molekulargewicht im Bereich von und einschließlich 15 kDa bis 80 kDa ist;
x 1 ist;
und wobei die PTH-Verbindung eine Rest-PTH-Aktivität hat im Vergleich zur Aktivität des entsprechenden freien PTHs von weniger als 1%.

2. Die pharmazeutische Zusammensetzung nach Anspruch 1, wobei die subkutane Verabreichung mit einer Pen-Vorrichtung erfolgt.

3. Die pharmazeutische Zusammensetzung nach Anspruch 1 oder 2, wobei das Hoch-zu-Tiefpunktverhältnis weniger als 3 ist.

4. Die pharmazeutische Zusammensetzung nach einem der Ansprüche 1 bis 3, wobei die Rest-PTH-Aktivität weniger als 0.1% ist.

5. Die pharmazeutische Zusammensetzung nach einem der Ansprüche 1 bis 4, wobei die pharmazeutische Zusammensetzung einen pH im Bereich von und einschließlich pH 3 bis pH 8 hat.

6. Die pharmazeutische Zusammensetzung nach einem der Ansprüche 1 bis 5, wobei die pharmazeutische Zusammensetzung einen pH im Bereich von und einschließlich pH 4 bis pH 5 hat.

7. Die pharmazeutische Zusammensetzung nach einem der Ansprüche 1 bis 6, wobei -D die Sequenz der SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:107, SEQ ID NO:108, SEQ ID NO:109, SEQ ID NO:110, SEQ ID NO:111, SEQ ID NO:112, SEQ ID NO:113, SEQ ID NO:114 oder SEQ ID NO:115 hat.

8. Die pharmazeutische Zusammensetzung nach einem der Ansprüche 1 bis 7, wobei -D die Sequenz der SEQ ID NO:51 hat.

9. Die pharmazeutische Zusammensetzung nach einem der Ansprüche 1 bis 8, wobei -L²- ausgewählt ist aus der Gruppe bestehend aus -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀-Alkyl, C₂₋₅₀-Alkenyl und C₂₋₅₀-Alkynyl; wobei -T-, C₁₋₅₀-Alkyl, C₂₋₅₀-Alkenyl und C₂₋₅₀-Alkynyl optional substituiert sind mit einem oder mehreren -R^{y2}, die gleich oder unterschiedlich sind, und wobei C₁₋₅₀-Alkyl, C₂₋₅₀-Alkenyl und C₂₋₅₀-Alkynyl optional unterbrochen sind durch eine oder mehrere Gruppen ausgewählt aus der Gruppe bestehend aus -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})- und -OC(O)N(R^{y3})-;
-R^{y1} und -R^{y1a} unabhängig voneinander ausgewählt sind aus der Gruppe bestehend aus -H, -T, C₁₋₁₀-Alkyl, C₂₋₁₀-Alkenyl und C₂₋₁₀-Alkynyl; wobei -T, C₁₋₁₀-Alkyl, C₂₋₁₀-Alkenyl und C₂₋₁₀-Alkynyl optional substituiert sind mit einem oder mehreren -R^{y2}, die gleich oder unterschiedlich sind, und wobei C₁₋₁₀-Alkyl, C₂₋₁₀-Alkenyl und C₂₋₁₀-Alkynyl optional unterbrochen sind durch eine oder mehrere Gruppen ausgewählt aus der Gruppe bestehend aus -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})- und -OC(O)N(R^{y4})-;
jedes T unabhängig ausgewählt ist aus der Gruppe bestehend aus Phenyl, Naphthyl, Indenyl, Indanyl, Tetralinyl, C₃₋₁₀-Cycloalkyl, 3- bis 10-gliedrigem Heterocyclyl, 8- bis 11-gliedrigem Heterobicyclyl, 8- bis 30-gliedrigem Carbopolycyclyl und 8- bis 30-gliedrigem Heteropolycyclyl; wobei jedes T unabhängig optional substituiert ist mit einem oder mehreren -R^{y2}, die gleich oder unterschiedlich sind;
-R^{y2} ausgewählt ist aus der Gruppe bestehend aus Halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}) und C₁₋₆-Alkyl; wobei C₁₋₆-Alkyl optional substituiert ist mit einem oder mehreren Halogen, die gleich oder unterschiedlich sind; und jedes -R^{y3}, -R^{y3a}, -R^{y4}, -R^{y4a}, -R^{y5}, -R^{y5a} und -R^{y5b} unabhängig voneinander ausgewählt ist aus der Gruppe bestehend aus -H und C₁₋₆-Alkyl; wobei C₁₋₆-Alkyl optional substituiert ist mit einem oder mehreren Halogen, die gleich oder unterschiedlich sind.

10. Die pharmazeutische Zusammensetzung nach einem der Ansprüche 1 bis 9, wobei -L²- ein Molekulargewicht im Bereich von 14 g/mol bis 750 g/mol hat.

11. Die pharmazeutische Zusammensetzung nach einem der Ansprüche 1 bis 10, wobei -Z ein Molekulargewicht von ungefähr 40 kDa hat.

## Revendications

1. Composition pharmaceutique comprenant un composé PTH, dans laquelle après une administration sous-cutanée à un primate non humain, le profil pharmacocinétique du composé PTH présente un rapport de pic sur creux de moins de 4 dans un intervalle d'injection, qui est 24 heures, à l'état stable et le composé PTH étant un composé soluble dans l'eau de formule (Ia) ou un de ses sels pharmaceutiquement acceptables 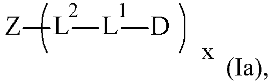
-D étant un groupement PTH ;
-L¹- étant de formule (IIb-iii') 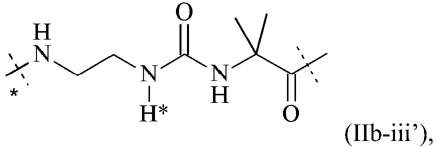
la ligne en pointillés indiquant la fixation au groupe fonctionnel amine N-terminale de -D par une liaison amide ;
la ligne en pointillés marquée par l'astérisque indiquant une fixation de -L²-Z ; et
-L¹- étant facultativement substitué, à condition que l'hydrogène marqué par l'astérisque dans la formule (IIb-iii') ne soit pas remplacé par un substituant ;
-L²- étant une liaison chimique simple ou un fragment espaceur ;
-Z étant un groupement support à base de PEG ramifié, soluble dans l'eau, avec un poids moléculaire allant de 15 kDa à 80 kDa inclus ;
x étant 1 ;
et le composé PTH ayant une activité résiduelle de PTH de moins de 1% par rapport à l'activité de PTH de la PTH libre correspondante.

2. Composition pharmaceutique selon la revendication 1, l'administration sous-cutanée ayant lieu avec un dispositif de type stylo.

3. Composition pharmaceutique selon la revendication 1 ou 2, le rapport de pic sur creux étant inférieur à 3.

4. Composition pharmaceutique selon l'une quelconque des revendications 1 à 3, l'activité résiduelle de PTH étant inférieur à 0,1%.

5. Composition pharmaceutique selon l'une quelconque des revendications 1 à 4, la composition pharmaceutique ayant un pH allant de pH 3 à pH 8 inclus.

6. Composition pharmaceutique selon l'une quelconque des revendications 1 à 5, la composition pharmaceutique ayant un pH allant de pH 4 à pH 5 inclus.

7. Composition pharmaceutique selon l'une quelconque des revendications 1 à 6, -D ayant la séquence de SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:107, SEQ ID NO:108, SEQ ID NO:109, SEQ ID NO:110, SEQ ID NO:111, SEQ ID NO:112, SEQ ID NO:113, SEQ ID NO:114 ou SEQ ID NO:115.

8. Composition pharmaceutique selon l'une quelconque des revendications 1 à 7, -D ayant la séquence de SEQ ID NO:51.

9. Composition pharmaceutique selon l'une quelconque des revendications 1 à 8, -L²- étant choisi dans le groupe constitué par -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, alkyle en C₁₋₅₀, alcényle en C₂₋₅₀ et alcynyle en C₂₋₅₀ ; -T-, alkyle en C₁₋₅₀, alcényle en C₂₋₅₀ et alcynyle en C₂₋₅₀ étant facultativement substitués par un ou plusieurs -R^{y2}, qui sont identiques ou différents, et alkyle en C₁₋₅₀, alcényle en C₂₋₅₀ et alcynyle en C₂₋₅₀ étant facultativement interrompus par un ou plusieurs groupes choisis dans le groupe constitué par -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})- et -OC(O)N(R^{y3})- ;
-R^{y1} et -R^{y1a} étant indépendamment choisis dans le groupe constitué par -H, -T, alkyle en C₁₋₁₀, alcényle en C₂₋₁₀ et alcynyle en C₂₋₁₀ ; -T, alkyle en C₁₋₁₀, alcényle en C₂₋₁₀ et alcynyle en C₂₋₁₀ étant facultativement substitués par un ou plusieurs -R^{y2}, qui sont identiques ou différents, et alkyle en C₁₋₁₀, alcényle en C₂₋₁₀ et alcynyle en C₂₋₁₀ étant facultativement interrompus par un ou plusieurs groupes choisis dans le groupe constitué par -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})- et -OC(O)N(R^{y4})- ;
chaque T étant indépendamment choisi dans le groupe constitué par phényl, naphthyle, indényle, indanyle, tétralinyle, cycloalkyle en C₃₋₁₀, hétérocyclyle de 3 à 10 chaînons, hétérobicyclyle de 8 à 11 chaînons, carbopolycyclyle de 8 à 30 chaînons et hétéropolycyclyle de 8 à 30 chaînons ; chaque T étant indépendamment et facultativement substitué par un ou plusieurs -R^{y2}, qui sont identiques ou différents ;
-R^{y2} étant choisi dans le groupe constitué d'halogène, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}) et alkyle en C₁₋₆ ; alkyle en C₁₋₆ étant facultativement substitué par un ou plusieurs halogènes, qui sont identiques ou différents ; et
chaque -R^{y3}, -R^{y3a}, -R^{y4}, -R^{y4a}, -R^{y5}, -R^{y5a} et -R^{y5b} étant indépendamment choisi dans le groupe constitué par -H et alkyle en C₁₋₆ ; alkyle en C₁₋₆ étant facultativement substitué par un ou plusieurs halogènes, qui sont identiques ou différents.

10. Composition pharmaceutique selon l'une quelconque des revendications 1 à 9, -L²- ayant un poids moléculaire allant de 14 g/mol à 750 g/mol inclus.

11. Composition pharmaceutique selon l'une quelconque des revendications 1 à 10, -Z ayant un poids moléculaire d'environ 40 kDa.
